(19) Europäisches Patentamt
European Patent Office
Office européen des brevets

(11) EP 2 493 477 B1

(12) EUROPEAN PATENT SPECIFICATION

(45) Date of publication and mention of the grant of the patent:
**16.10.2013 Bulletin 2013/42**

(21) Application number: **10776863.2**

(22) Date of filing: **26.10.2010**

(51) Int Cl.:
***A61K 31/55** (2006.01)* ***A61K 9/16** (2006.01)*
***A61K 9/20** (2006.01)* ***A61K 9/28** (2006.01)*
***A61K 31/404** (2006.01)*

(86) International application number:
**PCT/US2010/054022**

(87) International publication number:
**WO 2011/056532 (12.05.2011 Gazette 2011/19)**

# (54) BAZEDOXIFENE FORMULATIONS WITH ANTIOXIDANTS

BAZEDOXIFENFORMULIERUNGEN MIT ANTIOXIDATIONSMITTELN

PRÉPARATIONS DE BAZÉDOXIFÈNE AVEC ANTIOXYDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2009 US 255216 P**

(43) Date of publication of application:
**05.09.2012 Bulletin 2012/36**

(73) Proprietor: **Wyeth LLC**
**Madison, NJ 07940 (US)**

(72) Inventors:
- **AGRAWAL, Anjali**
  **Nyack**
  **New York 10960 (US)**
- **CHATLAPALLI, Ramarao S.**
  **Hopewell Junction**
  **New York 12533 (US)**
- **NAGI, Arwinder S.**
  **Thiells**
  **New York 10984 (US)**
- **VAN PELT, Lawrence**
  **Garnerville**
  **New York 10923 (US)**
- **CHIRRA, Srinivas**
  **North Brunswick**
  **New Jersey 08902 (US)**

(74) Representative: **Pfizer**
**European Patent Department**
**23-25 avenue du Docteur Lannelongue**
**75668 Paris Cedex 14 (FR)**

(56) References cited:
**WO-A1-2009/102773** **WO-A2-02/03987**
**WO-A2-2007/002823** **WO-A2-2008/067387**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## TECHNICAL FIELD

The disclosure is directed generally to the field of pharmaceutical compositions. More specifically, the disclosure relates to pharmaceutical compositions containing bazedoxifene and an antioxidant, as well as methods of enhancing dissolution stability and/or bioavailability of bazedoxifene in such compositions. In certain embodiments, the compositions include a core comprising conjugated estrogens, and at least one coating comprising bazedoxifene, or a pharmaceutically acceptable salt thereof, and at least one of vitamin E, vitamin E TPGS, propyl gallate, citric acid, and BHA/BHT, substantially free of ascorbic acid.

## BACKGROUND

Menopause is generally defined as the last natural menstrual period and is characterized by the cessation of ovarian function, leading to the substantial diminution of circulating estrogen in the bloodstream. Menopause is usually identified, in retrospect, after 12 months of amenorrhea. It is usually not a sudden event, but is often preceded by a time of irregular menstrual cycles prior to eventual cessation of menses. Following the cessation of menstruation, the decline in endogenous estrogen concentrations is typically rapid. There is a decrease in serum estrogens from circulating levels ranging from 40-250 pg/mL of estradiol and 40-170 pg/mL of estrone during ovulatory cycles to less than 15 pg/mL of estradiol and 30 pg/mL of estrone in postmenopausal women.

As these estrogens decline during the time preceding (perimenopause) and following the menopause (postmenopause), various physiological changes may result, including vulvar and vaginal atrophy causing vaginal dryness, pruritus and dyspareunia, and vasomotor instability manifested as hot flushes. Other menopausal disturbances may include depression, insomnia, and nervousness. The long- term physiologic effects of postmenopausal estrogen deprivation may result in significant morbidity and mortality due to increase in the risk factors for cardiovascular disease and osteoporosis. Menopausal changes in blood lipid levels, a major component of the pathogenesis of coronary heart disease (CHD), may be precursors to increased incidence of ischemic heart disease, atherosclerosis, and other cardiovascular disease. A rapid decrease in bone mass of both cortical (spine) and trabecular (hip) bone can be seen immediately after the menopause, with a total bone mass loss of 1% to 5% per year, continuing for 10 to 15 years.

Estrogen replacement therapy (ERT) is beneficial for symptomatic relief of hot flushes and genital atrophy and for prevention of postmenopausal osteoporosis. ERT has been recognized as an advantageous treatment for relief of vasomotor symptoms. Long term ERT can prevent osteoporosis because it decreases bone loss, reduces spine and hip fracture, and prevents loss of height. In addition, ERT is effective in increasing high density lipoprotein-cholesterol (HDL-C) and in reducing low density lipoprotein cholesterol (LDL-C), affording possible protection against CHD. ERT also can provide antioxidant protection against free radical-mediated disorders or disease states. Estrogens have also been reported to confer neuroprotection, and inhibit neurodegenerative disorders, such as Alzheimer's disease (see U.S. Pat. No. 5,554,601, which is herein incorporated by reference in its entirety).

The normal protocol for ERT calls for estrogen supplementation using such formulations containing estrone, estriol, ethynyl estradiol or conjugated estrogens isolated from natural sources (e.g., Premarin® conjugated estrogens from Wyeth). In some patients, therapy may be contraindicated due to the proliferative effects of unopposed estrogens on uterine tissue. This proliferation is associated with increased risk for endometriosis and/or endometrial cancer. The effects of unopposed estrogens on breast tissue are less clear, but are of some concern. Accordingly, one trend has been towards the development of low dose treatment regimen that minimizes the adverse effects of ERT.

Another approach has been to administer a progestin, either sequentially or in combination, with the estrogen. There are extensive clinical data showing that the relative risk of endometrial cancer can be reduced by the addition of a progestin to ERT. The addition of a progestin to estrogen therapy can help prevent estrogen-induced endometrial proliferation. With appropriate doses of daily estrogen and progestin, combined estrogen replacement therapy has been shown to be effective in relieving vaginal atrophy and vasomotor symptoms, preventing postmenopausal osteoporosis, and reducing the risk of endometrial cancer by prevention of endometrial hyperplasia.

A third approach to minimize the adverse effects of ERT is the use of selective estrogen receptor modulators (SERMs) in conjunction with ERT. SERMs are a class of compounds that demonstrate an affinity for estrogen receptors (ER) but show tissue selective estrogenic effects. An example of a SERM is bazedoxifene (1- [4- (2- azepan- 1- yl-ethoxy)- benzyl]- 2- (4- hydroxy- phenyl)- 3- methyl- 1H- indol- 5- ol; BZ), or a pharmaceutically acceptable salt thereof, such as bazedoxifene acetate (BZA), having the chemical formula shown below:

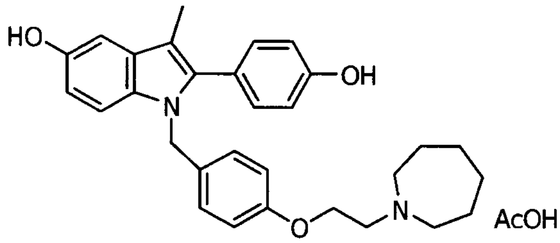

Bazedoxifene has been reported to prevent bone loss and protect the cardiovascular system and reduce or eliminate the negative effects on the uterus and breast (including potential risks of uterine and breast cancers). Consistent with its classification as a SERM, bazedoxifene demonstrates little or no stimulation of uterine response in preclinical models of uterine stimulation. Conversely, bazedoxifene demonstrates an estrogen agonist-like effect in preventing bone loss and reducing cholesterol in an ovariectomized rat model of osteopenia. In an MCF-7 cell line (human breast cancer cell line), bazedoxifene behaves as an estrogen antagonist. These data demonstrate that bazedoxifene is estrogenic on bone and cardiovascular lipid parameters and antiestrogenic on uterine and mammary tissue and thus has the potential for treating a number of different diseases or disease-like states wherein the estrogen receptor is involved.

U.S. Pat. Nos. 5,998,402 and 6,479,535 report the preparation of bazedoxifene, each of which is herein incorporated by reference in its entirety. The synthetic preparation of bazedoxifene has also appeared in the general literature. See, for example, Miller et al., J. Med. Chem., 2001, 44, 1654-1657, which is herein incorporated by reference in its entirety. Description of the drug's biological activity has also appeared in the general literature (*e.g.*, Miller, et al., Drugs of the Future, 2002, 27(2), 117-121, which is herein incorporated by reference in it*s entirety). In addition, pharmaceutical compositions containing bazedoxifene are* described, *e.g.*, in WO 02/03987, which is herein incorporated by reference in its entirety. Further, co-administration of bazedoxifene with estrogens is disclosed, e.g., in U.S. Pat. No. 6,479,535 and U.S. Publication Nos. 2007/0003623, 2008/0175905 and 2008/0175908, each of which is herein incorporated by reference in its entirety.

Polymorphic Form A of bazedoxifene is disclosed in U.S. Publication No. 2005/0227965 while polymorphic Form B of bazedoxifene is disclosed in U.S. Publication No. 2005/0250762, each of which is herein incorporated by reference in its entirety. Methods of preparing polymorphic Form A of bazedoxifene are also disclosed in commonly assigned and co-pending United States Patent Application Serial Nos. 12/369,104 and 12/369,315 filed on February 11, 2009, each of which is herein incorporated by reference in its entirety. Form A has higher solubility in both aqueous and organic solvent systems than Form B. This can be advantageous in formulations or doses where the solubility of the particular composition is of concern. For example, higher solubility can influence bioavailability, which can affect biological absorption and distribution of the drug, as well as facilitate formulation in liquid carriers. However, Form A is the kinetic (or meta-stable) polymorph while Form B is the thermodynamically more stable polymorph. Form A can easily convert to Form B upon contact with a solvent or solvent mixture (*e.g.*, ethyl acetate and ethanol), which presents a challenge to the preparation of pure Form A that is substantially free of Form B. Further, under various conditions, and over time, Form A can convert to Form B.

It has been observed that tablet compositions containing bazedoxifene acetate, ascorbic acid and hydroxypropyl methylcellulose show a decrease in dissolution stability on long term storage. Dissolution stability refers to retention of the dissolution characteristics of a solid oral dosage form of a drug from the time of manufacture up to its expiration date. Dissolution stability can impact quality control, regulatory compliance, and/or bioavailability of the drug. A shift in the dissolution profile of an oral drug product can impact the rate and the amount of the drug available for absorption, and therefore may influence the therapeutic efficacy of the drug. Accordingly, it is desirable that the dissolution characteristics of the solid dosage form remain substantially unchanged over its shelf-life. Thus, there is a need for pharmaceutical compositions of bazedoxifene having enhanced dissolution stability.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

## SUMMARY

Disclosed herein are pharmaceutical compositions containing bazedoxifene and an antioxidant. In certain embodiments, the compositions include a core comprising conjugated estrogens, and at least one coating comprising bazedoxifene, or a pharmaceutically acceptable salt thereof, and at least one of vitamin E, vitamin E TPGS, propyl gallate, citric acid, and BHA/BHT, substantially free of ascorbic acid.

In one aspect, the disclosure provides a method of enhancing dissolution stability of bazedoxifene in a pharmaceutical composition comprising bazedoxifene, or a pharmaceutically acceptable salt thereof, the method comprising formulating the pharmaceutical composition to comprise at least one of vitamin E and vitamin E TPGS, wherein the pharmaceutical composition is substantially free of ascorbic acid.

In another aspect, the disclosure provides a method of enhancing dissolution stability of bazedoxifene, or a pharmaceutically acceptable salt thereof, in a pharmaceutical composition, the method comprising preparing a bazedoxifene suspension substantially free of ascorbic acid by adding a wetting agent to water and mixing until the wetting agent is substantially dispersed in the water, forming a suspension; adding a binder to the suspension and mixing until the binder is substantially dispersed in the suspension; adding a filler to the suspension and mixing until the filler is substantially dissolved in the suspension; adding at least one of vitamin E and vitamin E TPGS to the suspension and mixing until the at least one of vitamin E and vitamin E TPGS is substantially dissolved in the suspension; and adding bazedoxifene, or a pharmaceutically acceptable salt thereof, to the suspension and mixing until the bazedoxifene is substantially dispersed in the suspension; providing a core comprising conjugated estrogens; coating the core with at least one coating comprising the bazedoxifene suspension; and drying the at least one coating to produce a pharmaceutical composition having a bazedoxifene coating substantially free of ascorbic acid and having enhanced dissolution stability relative to a pharmaceutical composition having a coating comprising bazedoxifene and ascorbic acid.

In certain embodiments, the bazedoxifene coating comprises from about 0.01 % to about 10% by weight of the coating as the at least one of vitamin E and vitamin E TPGS.

In certain embodiments, the bazedoxifene coating comprises from about 10% to about 40% by weight of the coating as the bazedoxifene.

In certain embodiments, the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosillicates, calcium phosphate, and metal carbonate; the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, copovidone, xanthan gum, and guar gum; and the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides.

In one embodiment, the filler is sucrose.

In one embodiment, the binder is hydroxypropyl methylcellulose.

In one embodiment, the wetting agent is sucrose palmitic acid ester.

In certain embodiments, the filler is sucrose, the binder is hydroxypropyl methylcellulose, and the wetting agent is sucrose palmitic acid ester.

In another aspect, the disclosure provides a method of reducing interactions of at least one of bazedoxifene and hydroxypropyl methylcellulose with at least one of ascorbic acid and one or more degradant products of ascorbic acid in a pharmaceutical composition comprising bazedoxifene, or a pharmaceutically acceptable salt thereof, hydroxypropyl methylcellulose, and ascorbic acid, the method comprising replacing part or all of said ascorbic acid with at least one of vitamin E and vitamin E TPGS in the pharmaceutical composition.

In another aspect, the disclosure provides a method of enhancing bioavailability of bazedoxifene in a pharmaceutical composition comprising bazedoxifene, or a pharmaceutically acceptable salt thereof, the method comprising formulating the pharmaceutical composition to comprise at least one of vitamin E and vitamin E TPGS, wherein the pharmaceutical composition is substantially free of ascorbic acid.

In another aspect, the disclosure provides a pharmaceutical composition comprising bazedoxifene, or a pharmaceutically acceptable salt thereof, a filler, a binder, a wetting agent, and at least one of vitamin E and vitamin E TPGS, wherein the pharmaceutical composition is substantially free of ascorbic acid, wherein the dissolution stability of bazedoxifene in the pharmaceutical composition is enhanced compared to the dissolution stability of bazedoxifene in a pharmaceutical composition comprising bazedoxifene and ascorbic acid.

In certain embodiments, the pharmaceutical composition comprises from about 10% to about 40% by weight as bazedoxifene.

In certain embodiments, the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosillicates, calcium phosphate, and metal carbonate; the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, copovidone, xanthan gum, and guar gum; and the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides.

In one embodiment, the filler is sucrose.

In one embodiment, the binder is hydroxypropyl methylcellulose.

In one embodiment, the wetting agent is sucrose palmitic acid ester.

In certain embodiments, the filler is sucrose, the binder is hydroxypropyl methylcellulose, and the wetting agent is sucrose palmitic acid ester.

In another aspect, the disclosure provides a pharmaceutical composition, comprising a core comprising conjugated estrogens; and at least one coating comprising bazedoxifene, or a pharmaceutically acceptable salt thereof, a filler, a binder, a wetting agent, and at least one of vitamin E and vitamin E TPGS, wherein the at least one coating is substantially free of ascorbic acid, and wherein the dissolution stability of bazedoxifene in the pharmaceutical composition is enhanced compared to the dissolution stability of bazedoxifene in a pharmaceutical composition comprising bazedoxifene and ascorbic acid.

In certain embodiments, the Pharmaceutical composition comprises from about 45% to about 80% by weight of the total composition as the core.

In certain embodiments, the core comprises from about 0.1 mg to about 1.25 mg conjugated estrogens.

In one embodiment, the pharmaceutically acceptable salt of bazedoxifene is bazedoxifene acetate.

In certain embodiments, the pharmaceutical composition comprises from about 3% to about 10% by weight of the total composition as bazedoxifene, from about 5% to about 30% by weight of the total composition as filler, from about 3% to about 10% by weight of the total composition as binder, from about 0.01% to about 2% by weight of the total composition as wetting agent, and from about 0.01% to about 2% by weight of the of the total composition as at least one of vitamin E and vitamin E TPGS.

In certain embodiments, the pharmaceutical composition comprises about 0.1 % or greater by weight of the at least one of vitamin E and vitamin E TPGS.

In certain embodiments, the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosillicates, calcium phosphate, and metal carbonate; the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, copovidone, xanthan gum, and guar gum; and the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides.

In one embodiment, the filler is sucrose.

In one embodiment, the binder is hydroxypropyl methylcellulose.

In one embodiment, the wetting agent is sucrose palmitic acid ester.

In certain embodiments, the filler is sucrose, the binder is hydroxypropyl methylcellulose, and the wetting agent is sucrose palmitic acid ester.

In another aspect, the disclosure provides a pharmaceutical composition comprising a core tablet comprising at least one conjugated estrogen, said core tablet forming from about 45% to about 80% by weight of the total composition; and an outer layer substantially free of ascorbic acid, comprising bazedoxifene acetate from about 4% to about 8% by weight of the total composition; sucrose from about 10% to about 20% by weight of the total composition; hydroxypropyl methylcellulose from about 4% to about 8% by weight of the total composition; sucrose palmitic acid ester from about 0.2% to about 0.6% by weight of the total composition; and at least one of vitamin E and vitamin E TPGS from about 0.1 % to about 2% to by weight of the total composition, provided that the total % of the composition by weight is 100%, wherein the dissolution stability of bazedoxifene acetate in the pharmaceutical composition is enhanced compared to the dissolution stability of bazedoxifene acetate in a pharmaceutical composition comprising bazedoxifene acetate and ascorbic acid.

In one embodiment, the pharmaceutical composition is packaged with an oxygen scavenger.

In another aspect, the disclosure provides a method of enhancing dissolution stability of bazedoxifene in a pharmaceutical composition comprising bazedoxifene, or a pharmaceutically acceptable salt thereof, the method comprising formulating the pharmaceutical composition to comprise at least one of propyl gallate, citric acid, and BHA/BHT, wherein the Pharmaceutical composition is substantially free of ascorbic acid.

In another aspect, the disclosure provides a pharmaceutical composition comprising bazedoxifene, or a pharmaceutically acceptable salt thereof, a filler, a binder, a wetting agent, and at least one of propyl gallate, citric acid, and BHA/BHT, wherein the pharmaceutical composition is substantially free of ascorbic acid, and wherein the dissolution

stability of bazedoxifene in the pharmaceutical composition is enhanced compared to the dissolution stability of bazedoxifene in a pharmaceutical composition comprising bazedoxifene and ascorbic acid.

## BRIEF DESCRIPTION OF THE DRAWINGS

The following figures are included for purposes of illustration and are not intended to be limiting.

FIGURE 1 shows the dissolution shift of BZA over time (1 month (Mnth), 4 months, and 6 months) in closed bottle, 40 °C/75% relative humidity (RH), with and without dessicant (Desc), for BZA/CE tablets containing 2 mg/tablet ascorbic acid in the BZA suspension, analyzed in acetic acid (AA)/Tween dissolution medium.

FIGURE 2 shows the dissolution shift of BZA over time in open dish (1 week (W), 2W, 4W) and closed bottle with dessicant (1 month), 40 °C/75% RH, for BZA/CE tablets containing 2 mg/tablet ascorbic acid, analyzed in AA/Tween dissolution medium.

FIGURE 3 shows the dissolution shift of BZA over time in open dish (1 W, 2W, 4W), 40 °C/75% RH, for BZA/CE tablets containing 2 mg/tablet ascorbic acid, analyzed in 0.02 N HCl dissolution medium.

FIGURE 4 shows the dissolution of BZA over time in open dish, 40 °C/75% RH, for BZA/CE tablets containing 1.0 mg/tablet vitamin E, analyzed in AA/Tween dissolution medium.

FIGURE 5 shows the dissolution of BZA over time in open dish, 40 °C/75% RH, for BZA/CE tablets containing 4.0 mg/tablet vitamin E TPGS, analyzed in AA/Tween dissolution medium.

FIGURE 6 shows the dissolution of BZA over time in open dish, 40 °C/75% RH, for BZA/CE tablets containing 0.5 mg/tablet propyl gallate, analyzed in 0.02 N HCl dissolution medium.

FIGURE 7 shows the dissolution of BZA over time in open dish, 40 °C/75% RH, for BZA/CE tablets containing 0.5 mg/tablet citric acid, analyzed in 0.02 N HCl dissolution medium.

FIGURE 8 shows the dissolution of BZA over time in open dish, 40 °C/75% RH, for BZA/CE tablets containing 1.0/0.5 mg/tablet butylated hydroxyanisole/butylated hydroxytoluene (BHA/BHT), analyzed in 0.02 N HCl dissolution medium.

FIGURE 9 shows the dissolution of BZA over time in open dish, 40 °C/75% RH, for BZA/CE tablets containing 2.0/0.2 mg/tablet ascorbic acid/calcium disodium ethylenediaminetetra acetic acid (EDTA), analyzed in 0.02 N HCl dissolution medium.

FIGURE 10 shows the dissolution of BZA over time in closed bottle, 40 °C/75% RH, with and without oxygen scavenger, for BZA/CE tablets containing 0.5 mg/tablet vitamin E, analyzed in AA/Tween dissolution medium.

FIGURE 11 shows the dissolution of BZA over time in closed bottle, 40 °C/75% RH, with and without oxygen scavenger, for BZA/CE tablets containing 1.0 mg/tablet vitamin E, analyzed in AA/Tween dissolution medium.

FIGURE 12 shows BZA dissolution stability in a closed bottle without dessicant at 12 months (M), room temperature (25 °C/60% RH) and 30 °C/75% RH, for BZA/CE tablets containing 1.0 mg/tablet 1.0 mg/tablet vitamin E, analyzed in AA/Tween dissolution medium.

FIGURE 13 shows BZA dissolution stability in a closed bottle without dessicant at 21 M, 25 °C/60% RH, for BZA/CE tablets containing 0.5 mg/tablet citric acid, analyzed in AA/Tween dissolution medium.

FIGURE 14 shows BZA dissolution stability in a closed bottle without dessicant at 16 M, 25 °C/60% RH, for BZA/CE tablets containing 1.0/0.5 mg/tablet BHA/BHT, analyzed in AA/Tween dissolution medium.

FIGURE 15 shows the chemical stability of BZA at 3 months in closed bottle, 40 °C/75% RH, for BZA/CE formulations containing 2 mg/tablet ascorbic acid and 1.0 mg/tablet vitamin E, with and without oxygen scavenger.

FIGURE 16 shows mean plasma concentration of BZA following single oral dose administration of BZA/CE tablets containing 2 mg/tablet ascorbic acid (Form A) or 1.0 mg/tablet vitamin E.

## DEFINITIONS

As used herein, the term "about" means plus or minus 10% of the value, unless otherwise indicated herein.

Cellulose gum, powdered cellulose, microcrystalline cellulose, and hydroxypropyl methylcellulose include, but are not limited to, those described in R. C. Rowe and P. J. Shesky, Handbook of Pharmaceutical Excipients, (Great Britain: Pharmaceutical Press; Washington, DC: American Pharmacists Association, 5th ed., 2006, which is herein incorporated by reference in its entirety).

Suitable powdered celluloses for use in the invention include, but are not limited to Arbocel®, Sanacel®, and Solka-Floc®.

Suitable microcrystalline celluloses include, but are not limited to, the Avicel® pH series, Celex™, Celphere™, Ceolus KG®, and Vivapur®. In certain embodiments, the microcrystalline cellulose is Avicel® pH200.

Hydroxypropyl methylcellulose is also known as Hypromellose or HPMC. Suitable Hydroxypropyl methylcelluloses include, without limitation, Benecel®MP643, Isopto® Tears; Methopt™; Poly-Tears ; Tears Naturale®, Methocel E™, Methocel F™, Methocel K™, and Pharmacoat®/Metolose®.

Starches include, but are not limited to, those described in R. C. Rowe and P. J. Shesky, *Handbook of Pharmaceutical Excipients.*

As used herein, the term "starch" refers to any type of natural or modified starch including, but not limited to, maize starch (also known as corn starch or maydis amylum), potato starch (also known as solani amylum), rice starch (also known as oryzae amylum), wheat starch (also known as tritici amylum), and tapioca starch. The term "starch" also refers to starches that have been modified with regard to molecular weight and branching. The term "starch" further refers to starches that have been chemically modified to attach chemical functionality such as carboxy, hydroxyl, hydroxyalkylene, or carboxyalkylene groups. As used herein, the term "carboxyalkylene" refers to a group of formula-alkylene- C (O) OH, or salt thereof. As used herein, the term "hydroxyalkylene" refers to a group of formula- alkylene- OH.

As used herein, the term "calcium phosphate" refers to monobasic calcium phosophate, dibasic calcium phosphate or tribasic calcium phosphate.

As used herein, the term "metal carbonate" refers to any metallic carbonate, including, but not limited to sodium carbonate, calcium carbonate, and magnesium carbonate, and zinc carbonate.

As used herein, the term "copovidone" refers to a copolymer of vinylpyrrolidone and vinyl acetate, wherein the vinyl acetate monomers may be partially hydrolyzed. Suitable copovidone polymers include, but are not limited to Kollidon® VA 64, Luviskol® VA, Plasdone® S-630, and Majsao® CT. For example, Plasdone® S-630 is a 60:40 copolymer of vinylpyrrolidone and vinyl acetate, and can be purchased from, *e.g.*, ISP (International Specialty Products), New Jersey, U.S.A.

As used herein, the term "fatty acid," employed alone or in combination with other terms, refers to an aliphatic acid that is saturated or unsaturated. In one embodiment, the fatty acid is a mixture of different fatty acids. In another embodiment, the fatty acid has between about eight to about thirty carbons on average. In yet another embodiment, the fatty acid has about eight to about twenty-four carbons on average. In yet another embodiment, the fatty acid has about twelve to about eighteen carbons on average. Suitable fatty acids include, but are not limited to, stearic acid, lauric acid, myristic acid, erucic acid, palmitic acid, palmitoleic acid, capric acid, caprylic acid, oleic acid, linoleic acid, linolenic acid, hydroxystearic acid, 12-hydroxystearic acid, cetostearic acid, isostearic acid, sesquioleic acid, sesqui-9-octadecanoic acid, sesquiisooctadecanoic acid, benhenic acid, isobehenic acid, and arachidonic acid, or mixtures thereof.

As used herein, the term "fatty acid ester" refers to a compound formed between a fatty acid and a hydroxyl containing compound. In one embodiment, the fatty acid ester is a polyoxyethylene sorbitan fatty acid ester. In another embodiment, the fatty acid ester is a sugar ester of fatty acid. In yet another embodiment, the fatty acid ester is a glyceride of fatty acid. In yet another embodiment, the fatty acid ester is a polyethoxylated fatty acid ester.

As used herein, the term "polyvinylpyrrolidone" refers to a polymer of vinylpyrrolidone. In one embodiment, the polyvinylpyrrolidone contains one or more additional polymerized monomers. In another embodiment, the additional polymerized monomer is a carboxy containing monomer. In yet another embodiment, the polyvinylpyrrolidone is povidone. In yet another embodiment, the polyvinylpyrrolidone has a molecular weight between 2500 and 3 million. In another embodiment, the polyvinylpyrrolidone is povidone K12, K17, K25, K30, K60, K90, or K120. Suitable polyvinylpyrrolidone polymers include, but are not limited to, the Kollidone® series (available from BASF) and the Plasdone® series (available from ISP).

As used herein, the term "docusate sodium" refers to dioctyl ester of sodium sulfosuccinate (bis- 2- ethylhexyl sodium sulfosuccinate) . As an effective anionic surfactant, docusate sodium is an excellent solubilizing, wetting, dispersing, or emulsifying agent, beneficial for use as a formulating aid for oral dosage forms.

As used herein, the term "quaternary ammonium amine compound" (also known as quaternary ammonium salts or quaternary ammonium cations) refers to a compound that contains at least one quaternary ammonium group, having the formula of $NR_4^+$ with R being alkyl groups. Each of the R groups may be same or different and any of the R groups may be connected to form a cyclic ring. Useful quaternary ammonium compounds include without limitation those that are capable of emulsifying, solubilizing, or suspending hydrophobic materials in water. Other suitable quaternary ammonium compounds include without limitation those that can enhance bioavailability of the active pharmacological agent when administered to the patient. Suitable quaternary ammonium compounds include, but are not limited to, 1, 2- dioleyl- 3- trimethylammonium propane, dimethyldioctadecylammonium bromide, N- [1- (1, 2- dioleyloxy) propyl]- N, N, N- trimethylammonium chloride, 1, 2- dioleyl- 3- ethylphosphocholine, or 3- β- [N- [(N', N'- dimethylamino) ethan] carbamoyl] cholesterol. Other suitable quaternary ammonium compounds include, but are not limited to, Stepanquat® 5ONF and 65NF (n- alkyl dimethyl benzyl ammonium chloride, Stepan Products, Northfield, IL) .

As used herein, the term "polyethoxylated fatty acid ester" refers to a monoester or diester, or mixture thereof, derived from the ethoxylation of a fatty acid. The polyethoyxylated fatty acid ester can contain free fatty acids and polyethylene glycol as well. Fatty acids useful for forming the polyethoxylated fatty acid esters include, but are not limited to, those described herein. Suitable polyethoxylated fatty acid esters include, but are not limited to, Emulphor® VT-679 (stearic acid 8.3 mole ethoxylate, available from Stepan Products, Northfield, IL), the Alkasurf® CO series (Alkaril Chemicals, Mississauga, Canada), macrogol 15 hydroxystearate, Solutol® HS15 (BASF, Florham Park, NJ), and the polyoxyethylene stearates listed in R. C. Rowe and P. J. Shesky, Handbook of Pharmaceutical Excipients.

As used herein, the term "polyethylene glycol" refers to a polymer containing ethylene glycol monomer units of formula- O- $CH_2$- $CH_2$- . Suitable polyethylene glycols may have a free hydroxyl group at each end of the polymer molecule, or may have one or more hydroxyl groups etherified with a lower alkyl, *e.g.*, a methyl group. Also suitable are derivatives of polyethylene glycols having esterifiable carboxy groups. In certain embodiments, polyethylene glycols can be polymers of any chain length or molecular weight, and can include branching. In one embodiment, the weight average molecular weight of the polyethylene glycol is from about 200 to about 9000. In another embodiment, the weight average molecular weight of the polyethylene glycol is from about 200 to about 5000. In yet another embodiment, the weight average molecular weight of the polyethylene glycol is from about 200 to about 900. In another embodiment, the weight average molecular weight of the polyethylene glycol is about 400.

Suitable polyethylene glycols include, but are not limited to polyethylene glycol-200, polyethylene glycol-300, polyethylene glycol-400, polyethylene glycol-600, and polyethylene glycol-900. The number following the dash in the name refers to the weight average molecular weight of the polymer. In one embodiment, the polyethylene glycol is polyethylene glycol-400. The weight average molecular weight is a way of describing the molecular weight of a polymer, and is calculated by

$$\bar{M}_w = \frac{\sum_i N_i M_i^2}{\sum_i N_i M_i}$$

where $N_i$ is the number of molecules of molecular weight $M_i$. The weight average molecular weight can be determined by light scattering, small angle neutron scattering (SANS), X-ray scattering, and sedimentation velocity. Suitable polyethylene glycols include, but are not limited to the Carbowax® and Carbowax® Sentry series (Dow Chemical Co., Midland, MI), the Lipoxol® series (Brenntag, Ruhr, Germany), the Lutrol® series (BASF, Florham Park, NJ), and the Pluriol® series (BASF, Florham Park, NJ).

As used herein, the term "polyethylene glycol-polypropylene glycol copolymer" refers to a copolymer that has both oxyethylene monomer units and oxypropylene monomer units. Suitable polyethylene glycol-polypropylene glycol copolymers for use in the invention can be of any chain length or molecular weight, and can include branching. The chain ends may have a free hydroxyl group or may have one or more hydroxyl groups etherified with a lower alkyl or carboxy group. The polyoxyethylene-polyoxypropylene copolymers can also include other monomers which were copolymerized and which form part of the backbone. For example, butylene oxide can be copolymerized with ethylene oxide and propylene oxide to form polyethylene glycol-polypropylene glycol copolymers useful in the present invention. In certain embodiments, the polyethylene glycol-polypropylene glycol copolymer is a block copolymer, wherein one block is polyoxyethylene and the other block is polyoxypropylene. Suitable polyethylene glycol-polypropylene glycol copolymer copolymers include, but are not limited to, Poloxamer 108, 124, 188, 217, 237, 238, 288, 338, 407, 101, 105, 122, 123, 124, 181, 182, 183, 184, 212, 231, 282, 331, 401, 402, 185, 215, 234, 235, 284, 333, 334, 335, and 403. Other suitable polyoxyethylene-polyoxypropylene copolymers include, but are not limited to, DowFax® Nonionic surfactants (Dow Chemical Co., Midland, MI), the DowFax® N-Series surfactants (Dow Chemical Co., Midland, MI), Lutrol® surfactants such as Lutrol® Micro 68 (BASF, Florham Park, NJ), and Synperonic® surfactants (Uniqema, Bromborough, UK).

As used herein, the term "polyoxyethylene castor oil derivatives " (or "polyethylene oxide castor oil derivatives") refers to a compound formed from the ethoxylation of castor oil, wherein at least one chain of polyethylene glycol is covalently bound to the castor oil. The castor oil may be hydrogenated or unhydrogenated. Synonyms for polyethylene oxide castor oil derivatives include, but are not limited to, polyoxyl castor oil, hydrogenated polyoxyl castor oil, macrogolglyceroli ricinoleas, macrogolglyceroli hydroxystearas, polyoxyl 35 castor oil, and polyoxyl 40 hydrogenated castor oil. Suitable polyethylene oxide castor oil derivatives include, but are not limited to, the Nikkol® HCO series (Nikko Chemicals Co. Ltd., Tokyo, Japan), such as Nikkol® HCO-30, HC-40, HC-50, and HC-60 (polyethylene glycol-30 hydrogenated castor oil, polyethylene glycol-40 hydrogenated castor oil, polyethylene glycol-50 hydrogenated castor oil, and polyethylene glycol-60 hydrogenated castor oil, Emulphor® EL-719 (castor oil 40 mole-ethoxylate, Stepan Products, Northfield, IL), the Cremophor® series (BASF, Florham Park, NJ), which includes Cremophor® RH40, RH60, and EL35 (polyethylene glycol-40 hydrogenated castor oil, polyethylene glycol-60 hydrogenated castor oil, and polyethylene glycol-35 hydrogenated castor oil, respectively), and the Eumulgin® RO and HRE series (Cognis PharmaLine, Monheim, Germany). Other suitable polyethylene oxide castor oil derivatives include those listed in R. C. Rowe and P. J. Shesky, Handbook of Pharmaceutical Excipients.

As used herein, the term, "polyoxyethylene sorbitan fatty acid ester" (or "polyethylene oxide sorbitan fatty esters") refers to a compound, or mixture thereof, derived from the ethoxylation of a sorbitan ester. As used herein, the term "sorbitan ester" refers to a compound, or mixture of compounds, derived from the esterification of sorbitol and at least one fatty acid. Fatty acids useful for deriving the polyethylene oxide sorbitan esters include, but are not limited to, those described herein. In one embodiment, the polyethylene oxide portion of the compound or mixture has about 2 to

about 200 oxyethylene units. In another embodiment, the polyethylene oxide portion of the compound or mixture has about 2 to about 100 oxyethylene units. In yet another embodiment, the polyethylene oxide portion of the compound or mixture has about 4 to about 80 oxyethylene units. In yet another embodiment, the polyoxyethylene portion of the compound or mixture has about 4 to about 40 oxyethylene units. In another embodiment, the polyethylene oxide portion of the compound or mixture has about 4 to about 20 oxyethylene units. Suitable polyethylene oxide sorbitan esters include, but are not limited to the Tween® series (Uniqema, Bromborough, UK), which includes Tween® 20 (POE(20) sorbitan monolaurate), 21 (POE(4) sorbitan monolaurate), 40 (POE(20) sorbitan monopalmitate), 60 (POE(20) sorbitan monostearate), 60K (POE(20) sorbitan monostearate), 61 (POE(4) sorbitan monostearate), 65 (POE(20) sorbitan tristearate), 80 (POE(20) sorbitan monooleate), 80K (POE(20) sorbitan monooleate), 81 (POE(5) sorbitan monooleate), and 85 (POE(20) sorbitan trioleate). As used herein, the abbreviation "POE" refers to polyethylene oxide. The number following the POE abbreviation refers to the number of oxyethylene repeat units in the compound. Other suitable polyethylene oxide sorbitan esters include the polyethylene oxide sorbitan fatty acid esters listed in R. C. Rowe and P. J. Shesky, Handbook of Pharmaceutical Excipients.

As used herein, the term "glycerides" refers to esters formed from glycerol and fatty acids. Glycerol has three hydroxyl functional groups, which can be esterified with one, two, or three fatty acids to form monoglycerides, diglycerides, and triglycerides.

As used herein, the term "polyglycolized glycerides" refers to the products formed from the esterification of polyethylene glycol, glycerol, and fatty acids; the transesterification of glycerides and polyethylene glycol; or the ethoxylation of a glyceride of a fatty acid. As used herein, the term "polyglycolized glycerides" can, alternatively or additionally, refer to mixtures of monoglycerides, diglycerides, and/or triglycerides with monoesters and/or diesters of polyethylene glycol. Polyglycolized glycerides can be derived from the fatty acids, glycerides of fatty acids, and polyethylene glycols described herein. The fatty ester side-chains on the glycerides, monoesters, or diesters can be of any chain length and can be saturated or unsaturated. The polyglycolized glycerides can contain other materials as contaminants or side-products, such as, but not limited to, polyethylene glycol, glycerol, and fatty acids. In certain embodiments, the polyglycolized glycerides are $C_8$-$C_{18}$ polyglycolized glycerides.

As used herein, unless otherwise modified by a specific salt, the term "bazedoxifene" means bazedoxifene or a pharmaceutically acceptable salt thereof.

As used herein, the term "conjugated estrogens" refers to estrogens that are joined together with another component-*e.g.*, sulfated or glycosylated. In certain embodiments, the conjugated estrogens are sodium sulfate conjugates, which contain a mixture of conjugated estrogens obtained exclusively from natural sources, occurring as the sodium salts of water-soluble estrogen sulfates blended to represent the average composition of material derived from pregnant mares' urine. The conjugated estrogens are a mixture of sodium estrone sulfate and sodium equilin sulfate, containing as concomitant components, as sodium sulfate conjugates, 17α-dihydroequilin, 17α-estradiol, and 17β-dihydroequilin.

"Conjugated estrogens" as used further herein includes both natural and synthetic conjugated estrogens, such as the compounds described in the United States Pharmacopia (USP 23), as well as other estrogens so considered by those skilled in the art. Further, "conjugated estrogens" refers to esters of such compounds, such as the sulfate esters, salts of such compounds, such as sodium salts, and esters of the salts of such compounds, such as sodium salts of a sulfate ester, as well as other derivatives known in the art.

Although CE are typically a mixture of estrogenic components, such as estrone and equilin, the core material may be formulated to either utilize such a mixture, or to include only selected or individual estrogenic components. These CE may be of synthetic or natural origin. Examples of synthetically produced estrogens include, inter alia, sodium estrone sulfate, sodium equilin sulfate, sodium 17α-dihydroequilin sulfate, sodium 17β-dihydroequilin sulfate, sodium 17α-estradiol sulfate, sodium 17β-estradiol sulfate, sodium equilenin sulfate, sodium 17α-dihydroequilenin sulfate, sodium 17β-dihydroequilenin sulfate, estropipate and ethinyl estradiol. The alkali metal salts of 8,9-dehydroestrone and the alkali metal salts of 8,9-dehydroestrone sulfate ester, as described in U.S. Pat. No. 5,210,081, which is herein incorporated by reference in its entirety. Naturally occurring CE are usually obtained from pregnant mare urine and then are processed and may be stabilized. Examples of such processes are set forth in U.S. Pat. Nos. 2,565,115 and 2,720,483, each of which is herein incorporated by reference in its entirety.

Many CE products are commercially available. Preferred among these is the naturally occurring CE product known as Premarin® (Wyeth, Madison, N.J.). Another commercially available CE product prepared from synthetic estrogens is Cenestin® (Duramed Pharmaceuticals, Inc., Cincinnati, Ohio). The specific CE dose may be any dosage required to achieve a specific therapeutic effect, and may vary depending on the specific treatment indicated, and on the specific CE included in the tablet.

## DETAILED DESCRIPTION

Disclosed herein are pharmaceutical compositions containing bazedoxifene and an antioxidant. In certain embodiments, the compositions include a core comprising conjugated estrogens, and at least one coating comprising bazedoxifene, or a pharmaceutically acceptable salt thereof, and at least one of vitamin E, vitamin E TPGS, propyl gallate, citric acid, and BHA/BHT, substantially free of ascorbic acid. In one aspect, the disclosure provides a method of enhancing dissolution stability and bioavailability of bazedoxifene in a pharmaceutical composition comprising bazedoxifene, or a pharmaceutically acceptable salt thereof, the method comprising formulating the pharmaceutical composition to comprise at least one of vitamin E and vitamin E TPGS, wherein the pharmaceutical composition is substantially free of ascorbic acid. In certain embodiments, the composition is packaged with an oxygen scavenger. In another aspect, the disclosure provides a method of enhancing dissolution stability of bazedoxifene in a pharmaceutical composition comprising bazedoxifene, or a pharmaceutically acceptable salt thereof, the method comprising formulating the pharmaceutical composition to comprise propyl gallate, citric acid, or BHA/BHT, wherein the pharmaceutical composition is substantially free of ascorbic acid.

Certain existing bazedoxifene compositions have contained, for example, 2 mg/tablet of ascorbic acid in a BZA coating layer that has been applied to CE cores. Such formulations were prepared by adding ascorbic acid to the BZA suspension during preparation of the suspension. It has been observed that tablet compositions containing BZA, ascorbic acid and hydroxypropyl methylcellulose (HPMC) show a decrease in bazedoxifene dissolution over long term storage. See, for example, Figure 1, which shows BZA dissolution shift over time in closed bottle, 40 °C/75% RH, for formulations containing ascorbic acid in the BZA coating layer.

Although ascorbic acid imparts excellent chemical stability to these formulations as an antioxidant, ascorbic acid has also been found to contribute to dissolution shifts, especially under accelerated storage conditions. While not being bound by any particular theory, it is believed that ascorbic acid and/or its degradants in the tablets may interact with bazedoxifene and/or HPMC, causing formulation changes surrounding bazedoxifene that result in dissolution shifts for tablets stored over time. Ascorbic acid lowers the pH of the BZA suspension and increases the solubility of BZA. Without being bound by any particular theory, potential ascorbic acid-associated mechanisms for BZA dissolution "slow down" could include formation of less soluble free BZ base due to weak association with ascorbic acid, formation of metastable amorphous BZA during coating due to increased solubility and later conversion of the metastable amorphous BZA to a stable crystalline form, and interaction of ascorbic acid with HPMC in the matrix leading to HPMC cross linking and resulting in slower dissolution or erosion of the BZA coat.

The disclosure relates to formulations having BZA and HPMC that employ alternate antioxidants. It has been found that replacing ascorbic acid with alternate antioxidants can reduce or eliminate shifts in bazedoxifene dissolution over time. Stability studies were performed to evaluate pharmaceutical compositions comprising BZA/CE tablets and alternate antioxidants, prepared substantially as described in Examples 1 and 2, placed in various temperature and humidity conditions.

Dissolution of BZA was evaluated in acetic acid (AA) / Tween® 80 and/or 0.02 N HCl as dissolution media (see Example 3), under aggressive storage conditions (open dish, 40 °C/ 75% RH), for formulations having various antioxidants. Figures 2 and 3 show the dissolution shifts of BZA over time for a BZA/CE formulation containing 2 mg/ tablet ascorbic acid, in AA/ Tween® (Figure 2) and 0.02 N HCl (Figure 3) . Dissolution of BZA in this formulation containing ascorbic acid as antioxidant in the BZA coating layer was notably decreased over time. Figures 4- 8 show the dissolution of BZA over time in aggressive storage conditions (open dish, 40 °C/ 75% RH), for BZA/CE formulations containing 1.0 mg/ tablet vitamin E, 4.0 mg/ tablet vitamin E TPGS, 0.5 mg/ tablet propyl gallate, 0.5 mg/ tablet citric acid, and 1.0/0.5 mg/ tablet BHA/BHT, respectively. Dissolution stability of bazedoxifene (evaluated in AA/ Tween®) was found to be enhanced in BZA/CE compositions having dl- alpha tocopherol (vitamin E; Figure 4) or vitamin E TPGS (dl- alpha tocopherol polyethylene glycol succinate; a water- soluble form of natural- source vitamin E prepared by esterifying d-alpha- tocopheryl acid succinate with polyethylene glycol; Figure 5) as antioxidant, as opposed to ascorbic acid (Figure 2) . Similarly, dissolution stability of bazedoxifene was also found to be enhanced in BZA/CE compositions having, for example, propyl gallate (Figure 6), citric acid (Figure 7), or BHA/BHT (Figure 8) as antioxidant, as opposed to ascorbic acid (Figure 3) . In contrast, dissolution stability of bazedoxifene was not enhanced in BZA/CE compositions having, for example, 2.0/0.2 mg/ tablet ascorbic acid/ calcium disodium EDTA (Figure 9) .

As shown in Figures 10 and 11, BZA/CE formulations having vitamin E at 0.5 and 1.0 mg/tablet provide good BZA dissolution stability upon storage in closed bottle, 40 °C/75% RH, with or without oxygen scavenger. In addition, no shifts in bazedoxifene dissolution were observed upon long term storage for formulations containing, for example, vitamin E (Figure 12), citric acid (Figure 13), or BHA/BHT (Figure 14).

Adequate chemical stability was provided by alternate antioxidants in these formulations as well. Chemical stability of BZA in BZA/CE tablets containing ascorbic acid or alternate antioxidants (see Example 4) is shown in Table 1.

Table 1

| Chemical Stability of BZA in BZA/CE Tablets with Various Antioxidants | | | | |
|---|---|---|---|---|
| **Antioxidant Type and Level** | **Storage condition** | **Cleavage** | **N-oxide** | **Total degradation** |
| 0 mg Ascorbic Acid (control) | Initial | 0.093 | 0.101 | 0.19 |
| | Open, 70 °C 4 days | 0.63 | 0.571 | 1.20 |
| 0.5 mg Ascorbic Acid | Initial | 0.031 | 0.137 | 0.17 |
| | Open, 70 °C 4 days | 0.172 | 0.481 | 0.65 |
| 1.0 mg Ascorbic Acid | Initial | 0.049 | 0.227 | 0.28 |
| | Open, 70 °C 4 days | 0.110 | 0.466 | 0.58 |
| 1.5 mg Ascorbic Acid | Initial | 0.036 | 0.097 | 0.13 |
| | Open, 70 °C 4 days | 0.046 | 0.199 | 0.25 |
| 2 mg Ascorbic Acid | Initial | 0.046 | 0.145 | 0.19 |
| | Open, 70 °C 4 days | 0.061 | 0.182 | 0.24 |
| 0.5 mg Citric Acid | Initial | 0.043 | 0.067 | 0.11 |
| | Open, 70 °C 4 days | 0.258 | 0.178 | 0.44 |
| 1.0/0.5 mg BHA/BHT | Initial | 0.040 | 0.060 | 0.10 |
| | Open, 70 °C 4 days | 0.228 | 0.255 | 0.48 |
| 0.5 mg Propyl Gallate | Initial | 0.042 | 0.050 | 0.09 |
| | Open, 70 °C 4 days | 0.236 | 0.236 | 0.47 |
| 0.5/0.05 mg Ascorbic Acid/EDTA | Initial | 0.066 | 0.165 | 0.23 |
| | Open, 70 °C 4 days | 0.124 | 0.019 | 0.14 |
| 0.5 mg Vit E | Initial | 0.055 | 0.077 | 0.13 |
| | Open, 70 °C 4 days | 0.236 | 0.269 | 0.51 |
| 1.0 mg Vit E | Initial | 0.069 | 0.108 | 0.18 |
| | Open, 70 °C 4 days | 0.215 | 0.226 | 0.44 |
| 4.0 mg Vit E TPGS | Initial | 0.049 | 0.078 | 0.13 |
| | Open, 70 °C 4 days | 0.173 | 0.225 | 0.40 |

As shown in Figure 15, the chemical stability provided by alternate antioxidants such as vitamin E can be enhanced with oxygen scavenger. Batches can be packaged with oxygen scavenger in, for example, bottles, blisters and bulk containers. Exemplary oxygen scavengers/oxygen absorbers include, but are not limited to, Stabilox® and Freshpax® manufactured by Multisorb, oxygen-scavenging polymer (OSP) by Chevron, oxygen absorbers by DPI and Impak, Ageless™-Z by Mitsubishi, UV activated oxygen scavengers by CSP, oxygen-scavenger (OS) by Cryovac, and Oxyfree™ 504 (iron and non-iron based) by Tianhua Tech. As shown in Table 1, chemical stability of BZA in BZA/CE tablets containing vitamin E TPGS is comparable to chemical stability of BZA in BZA/CE tablets containing vitamin E.

Further, it was surprisingly found that BZE/CE tablets having an alternate antioxidant such as vitamin E show a more rapid release rate and higher exposure of BZA *in vivo,* as compared to BZA/CE tablets having ascorbic acid as antioxidant. Bioavailability of bazedoxifene in BZA/CE tablets was studied in approximately 24 healthy postmenopausal women, to allow completion in at least 22 subjects. Blood samples were drawn at specified times for 24 hours, and plasma was separated and assayed for BZA and conjugated estrogens (estrone only) . As shown in Figure 16 and Table 2, the bioavailability of BZA in a BZA/CE formulation with 1 mg/ tablet vitamin E was higher than that of a formulation with 2 mg/ tablet ascorbic acid ("Form A" in Figure 16), both in terms of $C_{max}$ (highest concentration reached) and AUC (area under the curve; total exposure) . Specifically, $C_{max}$ and AUC increased about 43% and about 12% respectively when ascorbic acid (1 mg/ tablet) in the outer layer containing BZA was replaced with vitamin E (1 mg/ tablet) .

Table 2

| Mean plasma BZA pharmacokinetics following single oral dose administration of 20 mg/ 0.625 mg BZ/CE tablets in postmenopausal healthy volunteers | | | |
|---|---|---|---|
| | Parameter | $C_{max}$ (ng/mL) | AUC (ng·h/mL) |
| BZA/CE 2 mg/tablet ascorbic acid | Geometric mean | 4.48 | 60.6 |
| | CV, % | 58 | 54 |
| | | | |
| BZA/CE 1 mg/tablet vitamin E | Geometric mean | 6.4 | 72.5 |
| | CV, % | 35 | 43 |
| CV = coefficient of variation | | | |

The use of alternate antioxidants advantageously reduces or eliminates interactions between, for example, antioxidant (*e.g.*, ascorbic acid) and BZA and/or degradant product of antioxidant (*e.g.*, ascorbic acid) with HPMC. The use of alternate antioxidants also reduces solubility of BZA in a coating suspension used to make a coated BZA/CE tablet, thus potentially reducing the amorphous fraction of BZA that has the potential to crystallize into stable forms over time. While not being bound by any particular theory, it is believed that these factors result in the observed BZA dissolution stability and increased bioavailability due to faster erosion of BZA coat.

In summary, the use of alternate antioxidants such as vitamin E (dl-alpha tocopherol), vitamin E TPGS, propyl gallate, citric acid, and BHA/BHT in pharmaceutical compositions comprising bazedoxifene acetate and hydroxypropyl methylcellulose can reduce or eliminate the bazedoxifene dissolution slow down observed in formulations containing ascorbic acid as antioxidant, and can provide acceptable chemical stability/BZ degradation profiles. In addition, alternate antioxidants such as vitamin E can provide higher bioavailability, as compared to formulations containing ascorbic acid as antioxidant.

In addition to bazedoxifene and an antioxidant, the pharmaceutical compositions of the disclosure can further comprise at least one of a filler, a binder, and a wetting agent. In certain embodiments, the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosilicates, calcium phosphate, and metal carbonate; the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, co povidone, xanthan gum, and guar gum; and the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides. In certain embodiments, the filler is sucrose, the binder is hydroxypropyl methylcellulose, and the wetting agent is sucrose palmitic acid ester.

The Pharmaceutical compositions of the disclosure can also further comprise a core. In certain embodiments, the core comprises conjugated estrogens.

Antioxidants that may be used in the methods and pharmaceutical compositions of the disclosure include vitamin E, vitamin E TPGS, and propyl gallate, citric acid, and BHA/BHT. In certain embodiments, the compositions comprising alternate antioxidants are packaged with oxygen scavenger.

The pharmaceutical compositions described herein may be in the form of capsules, tablets (e.g., single- layer tablets, bi- layer tablets or tablet- in- tablets) and the like, each containing a predetermined amount of bazedoxifene as an active ingredient. In certain embodiments, the compositions may contain conjugated estrogens as an additional active ingredient. In certain embodiments, a conjugated estrogens (CE) core tablet is coated with a bazedoxifene acetate (BZA) suspension to produce a BZA/CE tablet formulation.

In at least some embodiments, bazedoxifene may be administered ranging from once every two days, to once per week. The dosage for a given dosing regimen can be given all at once or given multiple times on the same day. Based on individual patient needs, bazedoxifene may be administered every second day, every third day, every fourth day, every fifth day, every sixth day, or every seventh day (once weekly). The administration period may also be adjusted depending on the needs of the patient, and still be considered to be administered according to an extended dosing regimen.

For example, the dosage may be given once every other day, and then after medical follow-up be adjusted to be administered every third day, and eventually once weekly. The extended dosing regimen may be administered once weekly, where the weekly dosage is given on one day, either as a single dose, or divided into two or more doses during the same day.

In at least some embodiments, the daily dosage of bazedoxifene in humans is between about 5- 80 mg. When bazedoxifene is administered once weekly, the once per week dosage in at least some embodiments will be from about 3-15 times that of the daily dosage. Accordingly, in at least some embodiments the once weekly oral dosage may be between about 15 and 1200 mg given once per week; with the dosage being given in one or more doses during the administration day.

As described in U.S. Patent No. 5,998,402, which is herein incorporated by reference in its entirety, bazedoxifene and salts thereof are selective estrogen agonists with affinity for the estrogen receptor. Unlike other types of estrogen agonists, bazedoxifene and salts thereof are antiestrogenic in the uterus and can antagonize the trophic effects of estrogen agonists in uterine tissues. Accordingly, the pharmaceutical compositions described herein can find many uses related to treating disease states or syndromes associated with estrogen deficiency or excess of estrogen. In certain embodiments, the disclosure provides methods of treating a disease or disorder associated with estrogen deficiency or estrogen excess. Diseases and disorders associated with estrogen deficiency or estrogen excess include bone loss, osteoporosis, osteopenia, prostatic hypertrophy, male pattern baldness, vaginal and skin atrophy, acne, dysfunctional uterine bleeding, endometrial polyps, benign breast disease, uterine leiomyomas, adenomyosis, ovarian cancer, infertility, breast cancer, endometriosis, endometrial cancer, polycystic ovary syndrome, cardiovascular disease, contraception, Alzheimer's disease, cognitive decline and other CNS disorders, as well as certain cancers including melanoma, prostate cancer, cancers of the colon, CNS cancers, among others.

The pharmaceutical compositions described herein can also be used in methods of treatment for diseases or disorders which result from proliferation or abnormal development, actions or growth of endometrial or endometrial-like tissues. In certain embodiments, the disclosure provides methods of treating a disease or disorder associated with proliferation or abnormal development of endometrial tissues. Diseases and disorders associated with proliferation or abnormal development of endometrial tissues include endometrial polyps, endometriosis, and endometrial cancer.

The pharmaceutical compositions described herein can also be used in methods of inhibiting bone loss. Bone loss often results from an imbalance in an individual's formation of new bone tissues and the resorption of older tissues, leading to a net loss of bone. Such bone depletion can occur in a range of individuals, for example in post-menopausal women, women who have undergone bilateral oophorectomy, those receiving or who have received extended corticosteroid therapies, those experiencing gonadal dysgenesis, and those suffering from Cushing's syndrome. Special needs for bone, including teeth and oral bone replacement, can also be addressed using the compositions in individuals with bone fractures, defective bone structures, and those receiving bone-related surgeries and/or the implantation of prosthesis. In addition to the problems described above, in certain embodiments the compositions can be used in treatments for osteoarthritis, hypocalcemia, hypercalcemia, Paget's disease, osteomalacia, osteohalisteresis, multiple myeloma and other forms of cancer having deleterious effects on bone tissues.

The pharmaceutical compositions described herein can also be used in methods of lowering cholesterol and treating breast cancer. Additionally, the compositions can be used for treating perimenopausal, menopausal, or post-menopausal symptoms. In certain embodiments, the compositions can be used for contraception in pre-menopausal women, as well as hormone replacement therapy in post-menopausal women (such as for treating vasomotor disturbances such as hot flush) or in other estrogen deficiency states where estrogen supplementation would be beneficial. The compositions can also be used in disease states where amenorrhea is advantageous, such as leukemia, endometrial ablations, chronic renal or hepatic disease, or coagulation diseases or disorders.

The invention is further illustrated by the following examples. The examples are provided for illustrative purposes only. They are not to be construed as limiting the scope or content of the invention in any way.

## EXAMPLES

### Example 1

### Method of Preparing BZA/CE Tablets with Vitamin E

Pharmaceutical compositions of the disclosure were prepared as follows. Where exemplary process conditions (*e.g.*, ingredient, brand, amount, temperature, time) were identified, other suitable process conditions could also be used unless otherwise stated, as determined by one skilled in the art.

**I. Preparation of Conjugated Estrogens Inert Filled Tablets**

Granulation/Blending Process

Four sub-lots were granulated as follows:

1. Lactose monohydrate, microcrystalline cellulose, hypromellose 2208 (100,000 cps), CE desiccated with lactose and the remainder of lactose monohydrate were charged to a 1000L intermediate bulk container (IBC). The materials were transferred to a Collette UltimaGral and blended for about 6 minutes.

2. The blend in Step 1 was granulated by initiating the addition of purified water at a target rate of 17 kg/minute to the Collette granulator with continuous impeller at 85 rpm and intermittent choppers at 1500 rpm. The total time for water addition and wet massing was about 8 minutes.

3. The wet granulation was passed through a Quadro Comil using a 3.96 mm round screen and a target mill speed of 1200 rpm into a fluid bed dryer.

4. The granulation was dried in a fluid bed dryer at an inlet temperature set point of about 60 °C to achieve a target granulation loss on drying (LOD) moisture content of 2.0% ± 0.5%.

5. The dried granulation was passed through a Quadro Comil using a 1.27 mm grater screen in 4 phases with the following mill speeds, Phase 1 and 2 using 1200 rpm, Phase 3 at 1000 rpm and Phase 4 at 150 rpm, into a 2200L IBC.

6. The 2200L IBC with the four dry milled sub-lots of granulate was transferred to a Double Pedestal Bin Blender and blended for about 10 minutes at 8 rpm.

7. Magnesium stearate was added to the Step 6 blend, through a #12 screen and the resulting mixture was stirred for about 5 minutes.

Note: The quantity of magnesium stearate added should be adjusted on a per tablet basis based on the quantity of granulation to be blended.

Compression

1. The granulation was compressed into tablets using a Fette 2090i tablet press equipped with 0.412" x 0.225" x 0.034" (10.45 mm x 5.72 mm x 0.86 mm) oval shaped tooling at a target rate of 2250 - 4000 tpm with a "fillomatic" speed of 10 -120 rpm.

2. The compressed core tablet weight was approximately 120 mg.

The quantitative formula for compressed core CE tablets, 0.45 mg/tablet is shown in Table 3.

Table 3

| Ingredient | % WT/WT | Input/ Dosage Unit Input | Unit |
|---|---|---|---|
| C.E. desiccation with lactose @ 42.9 mg/g [a] | 8.74 | 10.4895 | mg |
| Lactose monohydrate, NF/EP, powder [a] | 48.51 | 58.2105 | mg |
| Hypromellose, USP, 2208 (100,000 cps) (K100M Prem, CR) | 27.5 | 33.00 | mg |
| Microcrystalline cellulose, NF/EP | 15.00 | 18.00 | mg |
| Water, USP/EP, purified[b] | | 30.00 | mg |

(continued)

| Ingredient | % WT/WT | Input/ Dosage Unit Input | Unit |
|---|---|---|---|
| Magnesium stearate, NF/EP vegetable code (Mallinckrodt 2257) [c] | 0.25 | 0.300 | mg |
| a. The quantity of lactose and CE desiccation with lactose should be adjusted if the active potency is not released at 42.9 mg/g.<br>b. Purified water, USP/EP used in the process did not appear in the final product.<br>c. Magnesium stearate may be added at the lubrication stage after cross blending of 4 sub-batches. | | | |

Other CE strengths may be used, for example 0.625 mg/tablet, or from 0.1-1.0 mg/tablet.

Preparation of Inert Filler Suspension

1. With low shear mixing, an appropriate amount of purified water was placed in a suitable jacketed mixing vessel equipped with low and high shear mixers, and the vessel temperature was set to 25 ± 5 °C.

2. With high shear mixing, hydroxypropyl cellulose was added to the vessel under vacuum and mixed until dispersed.

3. With high shear mixing, hypromellose 2910 (E6) was added to the vessel under vacuum and mixed until dispersed.

4. The resulting mixture was mixed with low and high shear mixing to obtain a uniform suspension.

5. With high and low shear mixing, hypromellose 2910 (E15) was added to the vessel and mixed until dispersed.

6. With low shear mixing, polyethylene glycol was added to the vessel manually and mixed until dispersed.

7. With high shear mixing, sucrose was added to the vessel under vacuum and mixed with high shear to obtain a uniform suspension.

8. With high shear mixing, microcrystalline cellulose was added to the vessel under vacuum and mixed until dispersed.

9. Mixing of the suspension was continued using low shear at 23 ± 5 °C until the filler suspension application was complete.

The formula for 1 Kg of inert filler suspension is shown in Table 4.

Table 4

| INGREDIENT | AMOUNT/BATCH (kg) |
|---|---|
| Sucrose, NF/EP | 0.0900 |
| Microcrystalline cellulose, NF/EP | 0.0144 |
| Hydroxypropyl Cellulose, klucel EF pharma, NF/EP | 0.0108 |
| Hypromellose 2910 USP/EP E6 | 0.0468 |
| Hypromellose, 2910, USP/EP E15 | 0.0117 |
| Polyethylene glycol 400, NF/EP | 0.0063 |

(continued)

| INGREDIENT | AMOUNT/BATCH (kg) |
|---|---|
| Water, USP/EP, purified | 0.8200 |
| Notes: The target amount of filler suspension applied was 0.500 g/tablet (90.0 mg solids at 18.0 % w/w). The actual amount of filler suspension manufactured and applied depended on manufacturing losses and coating efficiencies. Unused filler suspension should be destroyed. Density of filler suspension at room temperature was approximately 1.05 g/mL. | |

Tablet Coating Procedure

1. The conjugated estrogens compressed core tablets were loaded into a perforated coating pan.
2. Sufficient inert filler suspension was applied to the compressed cores to achieve an average filler solids weight gain of 90 mg (± 5 mg) above the average compressed core weight. (Interim alert level for individual filled tablets weights: 187-243 mg per tablet, n=300.)
3. Finished tablets were stored in containers lined with double polyethylene bags or equivalent with desiccant.
4. The final inert filled tablet weight was approximately 210 mg.

Note: The quantity of magnesium stearate added should be adjusted on a per tablet basis based on the quantity of granulation to be blended.

The quantitative formula for inert filled CE tablets is shown in Table 5.

Table 5

| Ingredient | % WT/WT | Input/ Dosage Unit Input | Unit |
|---|---|---|---|
| CE compressed core | 57.14 | 120 | mg |
| Sucrose, NF/EP | 21.43 | 45.0 | mg |
| Microcrystalline cellulose, NF/EP | 3.43 | 7.20 | mg |
| Hydroxypropyl cellulose, klucel EF pharma, NF/EP | 2.57 | 5.4 | mg |
| Hypromellose, 2910, USP/EP E6 | 11.14 | 23.4 | mg |
| Hypromellose, 2910, USP/EP E15 | 2.79 | 5.85 | mg |
| Polyethylene glycol 400, NF/EP | 1.5 | 3.15 | mg |
| Water, USP/EP, purified* | | 410 | mg |
| * Purified water, USP/EP, used in the process did not appear in the final product. | | | |

Notes: The final inert filled tablet weight was approximately 210 mg. Each batch contained 2 pan loads of 3.4 million tablets. Two inert filler suspensions were prepared to coat 2 pan loads. The inert filler ingredients included approximately 35% overage.

**II. Preparation of Bazedoxifene Acetate Filler Suspension**

Procedure

1. An appropriate amount of purified water was placed in a suitable mixing vessel equipped with low shear (Lightnin type) and high shear (Silverson type or equivalent) mixers. Weight of the water was recorded.

2. With the mixer turned on, water was heated to 65-85 °C (target 75 °C) and maintained at that temperature for

about 1 hour.

3. The speed of the mixer was adjusted to create a vortex without drawing air into the water. Speed of the mixer was recorded.

4. Sucrose palmitic acid ester was slowly added to the vortex and mixed until completely dispersed. High shear mixer was used as needed. Excessive foaming should be avoided. Time to complete dispersion was recorded (approximately 30-60 minutes). Any adjustments made to the mixers was recorded.

5. Hypromellose was slowly added to the vortex. Suspension temperature was maintained at 65-85 °C (target 75 °C). Speed of the mixer may be adjusted to obtain adequate mixing. However, introduction of air into the suspension should be minimized. All hypromellose was well dispersed without forming any clumps. High shear mixer was used as needed. Excessive foaming should be avoided. Time to complete dispersion was recorded (approximately 30-60 minutes). Any adjustments made to the mixers were recorded.

6. Suspension was cooled to 60-70 °C (target 65 °C). Sucrose was slowly added to the vortex, and mixing was continued until fully dissolved. Time to dissolve was recorded (approximately 30 minutes). Any adjustments made to the mixers were recorded.

7. The suspension was cooled to 23 - 27 °C (target 25 °C). The suspension was visually inspected to ensure that no particles remained.

8. After the temperature reached 23-27 °C (target 25 °C), dl-alpha tocopherol was slowly added, and mixing was continued until fully dissolved. Time to dissolve was recorded (approximately 15-30 minutes). Any adjustments made to the mixers were recorded.

9. BZA was slowly added to the vortex. Once added, high shear mixer was turned on and mixing was continued until fully dispersed. Excess foaming should be avoided. Time to complete dispersion was recorded (approximately 15-30 minutes). Contents were visually inspected to ensure that the suspension was uniformly dispersed. Any adjustments made to the mixers were recorded.

10. All the mixers were turned off and tank walls and mixer shaft were scraped. The mixers were restarted using previous settings and mix. Additional water was added if necessary (purified) to achieve theoretical weight, and mixing was continued for an additional 10-15 min. Contents were visually inspected to ensure that the suspension was uniformly dispersed. High shear mixer was turned off.

11. Mixing suspension was continued with the mixer at slow speed during application, while maintaining temperature at 23-27 °C (target 25 °C). All the mixer speeds were recorded.

Note: All the above steps can be carried out at ambient temperature if suitable equipment is not available. However, the mixing times may have to be extended in order for the excipients to dissolve or suspend. Sucrose palmitate, HPMC and sucrose may be added as a blend at 50-65 °C (target 60 °C) if the mixing vessel is under vacuum. The BZA, ascorbic acid and sucrose may be added as a blend at 23-27 °C (target 25 °C) if the mixing vessel is under vacuum.

The quantitative formula for 1 kg ofbazedoxifene acetate filler suspension (20% w/w solids) is shown in Table 6.

Table 6

| INGREDIENT | AMOUNT |
|---|---|
| Sucrose, NF | 100 g |
| Hypromellose, USP 2910, 3 cps | 48.0 g |
| Bazedoxifene acetate (@ 88.68% BZ free base) | 45.12* g |
| Sucrose palmitic acid ester | 2.50 g |
| dl-alpha tocopherol | 2.00 g |

(continued)

| INGREDIENT | AMOUNT |
|---|---|
| Water, USP, purified | 800 g |
| * The potency of bazedoxifene acetate may vary, and the amount in the formula should be adjusted accordingly with a corresponding adjustment in the amount of sucrose to maintain a 20% (w/w) solids content in the suspension. | |

**III. Preparation of Film/Color Coat Suspension**

Procedure

1. An appropriate amount of purified water at room temperature was added to a suitable size stainless steel container equipped with a low shear (Lightnin type) mixer. Weight of the water was recorded.

2. With the propeller in the center and as close to the bottom of the vessel as possible, the water was stirred to form a vortex without drawing air into the liquid.

3. Opadry Brown was steadily added directly to the vortex. (Opadry Brown was a formulated coating manufactured by Colorcon, West Point, Pennsylvania, U.S.A. and contained hydroxypropyl cellulose, hydroxypropyl methylcellulose, titanium dioxide and iron oxide.) Speed of low shear mixer was increased as needed to maintain vortex. Powder floatation on the water should be avoided.

4. After all Opadry Brown had been added, the mixer speed was reduced to eliminate vortex and gently mixed for approximately 45-60 minutes.

5. The mixing was continued gently during the coating application.

Note: Use the suspensions/solutions within 36 hours when stored at room temperature. The temperature of the water and the suspension may be maintained at a target of 25 °C (15-35 °C).

The quantitative formula for 1 kg of Film Coat suspension (12.5 % w/w solids) is shown below:

| INGREDIENT | AMOUNT |
|---|---|
| Opadry Brown 03B96519 | 125 g |
| Water USP, purified | 875 g |

An equivalent amount of Opadry Pink 03B 14899 (Colorcon, West Point, Pennsylvania, U.S.A.) or other color can be used in place of Opadry Brown.

**IV. Preparation of Clear/Gloss Coat Solution**

Procedure

1. An appropriate amount of purified water at room temperature was added to a stainless steel container equipped with a low shear (Lightnin type) mixer. Weight of the water was recorded.

2. With the propeller in the center and as close to the bottom of the vessel as possible, the water was stirred to form a vortex without drawing air into the liquid.

3. Opaglos 2 Clear (Colorcon, West Point, Pennsylvania, U.S.A.) was steadily added directly to the vortex. Speed of low shear mixer was increased as needed to maintain vortex. Powder floatation on the water should be avoided.

4. After all Opaglos 2 Clear had been added, the mixer speed was reduced to eliminate vortex and gently mixed for approximately 45-60 minutes.

5. The mixing was continued gently during the coating application.

Note: Use the suspensions/solutions within 36 hours when stored at room temperature. The temperature of the water and the suspension may be maintained at a target of 25 °C (15-35 °C).

The quantitative formula for 1 kg of Clear Coat Solution (5.0 % w/w solids) is shown below:

| INGREDIENT | AMOUNT |
|---|---|
| Opaglos 2 Clear 98Z19173 | 50.0 g |
| Water USP, purified | 950 g |

**V. Tablet Coating Procedure**

Procedure

1. The conjugated estrogen coated tablets (for example, as prepared in Section I above) were loaded into a perforated coating pan.

2. Sufficient bazedoxifene acetate filler suspension was applied to the step 1 CE filled tablets to achieve a total weight of 100 mg (± 2 mg) above the inert filled tablet weight. (Approximately 500 mg of bazedoxifene acetate filler suspension per tablet.)

3. Approximately 16 mg (± 1 mg) polymer color coat (Opadry Brown 03B96519) was applied to the tablets to achieve desired color. (Approximately 128 mg color suspension per tablet.)

4. Approximately 4 mg (± 1 mg) polymer gloss coat (Opaglos 2 Clear 98Z19173) was applied to the tablets to obtain satisfactory gloss. (Approximately 80 mg of suspension per tablet.)

5. The tablets were branded using Opacode black ink WB, NS-78-17821 (Colorcon, West Point, Pennsylvania, U.S.A.) on appropriate printing machine using a double line text print roller.

6. Finished tablets were stored, at room temperature, in containers lined with double polyethylene opaque bags with appropriate amount of dessicant. Alternately, finished tablets were stored at room temperature, in containers lined with a polyethylene opaque bag and an outer aluminum foil laminate bag with appropriate amount of oxygen scavenger between the two bags.

Note: Exemplary oxygen scavengers/oxygen absorbers include, but are not limited to, Stabilox® and Freshpax®, manufactured by Multisorb, oxygen-scavenging polymer (OSP) by Chevron, oxygen absorbers by DPI and Impak, Ageless™-Z by Mitsubishi, UV activated oxygen scavengers by CSP, oxygen-scavengers (OS) by Cryovac, and Oxyfree™ 504 (iron and non-iron based) by Tianhua Tech.

**VI. Formulation of BZA/CE Tablet with Vitamin E (dl-alpha Tocopherol)**

Tablets produced according to the above method had the following formulation as shown in Table 7.

Table 7

| | **Amount per unit** | **% wt/wt** |
|---|---|---|
| **Tablet Core** | | |
| Conjugated estrogens inert filled | 210 (1 Tab) mg | 63.64 |

(continued)

| | | |
|---|---|---|
| **Outer Laver (Active Coat)** | | |
| Sucrose, NF | 55.2 mg | 16.73 |
| Hypromellose, USP 2910, 3 cps | 20.0 mg | 6.06 |
| Bazedoxifene acetate, micronized (@88.68% bazedoxifene free base)^ | 22.56 mg | 6.84 |
| Sucrose palmitic acid ester | 1.25 mg | 0.38 |
| dl-alpha tocopherol | 1.00 mg | 0.30 |
| Water, USP purified* | N/A | |
| **Color Coat** | | |
| Opadrv Brown (03B96519) | 16.0 mg | 4.85 |
| Water, USP, purified* | N/A | |
| **Clear Coat** | | |
| Opaglos 2 Clear (98Z19173) | 4.00 mg | 1.21 |
| Water, USP, purified* | N/A | |
| **Branding** | | |
| Opacode black ink (WB, NS-78-17821) | 0.20 mg (trace solids) | N/A |
| Water, USP, purified* | N/A | |
| **TOTAL** | 330 mg | 100 |

* Used in the process, but did not appear in the final product.
^ The potency of bazedoxifene may vary; the amount in the formula should be adjusted accordingly with a corresponding adjustment in the amount of sucrose.
Note: The quantities of the ingredients per tablet represent theoretical amounts of coating solids applied. For elegance, the amount of water and coating ingredients used may vary.

Example 2

Method of Preparing BZA/CE Tablets with Vitamin E TPGS

Pharmaceutical compositions having vitamin E TPGS instead of vitamin E as antioxidant were prepared as in Example 1, with the following processes with respect to the preparation of bazedoxifene acetate filler suspension changed:

**II. Preparation of Bazedoxifene Acetate Filler Suspension**

1. An appropriate amount of purified water was placed in a suitable mixing vessel equipped with low shear (Lightnin type) and high shear (Silverson type or equivalent) mixers. Weight of the water was recorded.

2. With the mixer turned on, the water was heated to 65-85 °C (target 75 °C) and maintained at that temperature for about 1 hour.

3. The speed of the mixer was adjusted to create a vortex without drawing air into the water. The speed of the mixer was recorded.

4. Sucrose palmitic acid ester was slowly added to the vortex and mixed until completely dispersed. High shear mixer was used as needed. Excessive foaming should be avoided. Time to complete dispersion was recorded (approximately 30-60 minutes). Any adjustments made to the mixers were recorded.

5. Hypromellose was slowly added to the vortex. Suspension temperature was maintained at 65-85 °C (target 75

°C). The speed of the mixer may be adjusted to obtain adequate mixing. Introduction of air into the suspension should be minimized. All hypromellose was well dispersed without forming any clumps. High shear mixer was used as needed. Excessive foaming should be avoided. Time to complete dispersion was recorded (approximately 30-60 minutes). Any adjustments made to the mixers were recorded.

6. The resulting suspension was cooled to 60-70 °C (target 65 °C). Sucrose was slowly added to the vortex. Mixing was continued until fully dissolved. Time to dissolve was recorded (approximately 30 minutes). Any adjustments made to the mixers were recorded.

7. The weighed quantity of vitamin E TPGS was melted by heating to about 45 °C. Two kg of water was heated to 65-85 °C (target 75 °C) and molten vitamin E TPGS was slowly added to the water with continuous stirring using Lightnin mixer and mixed for approximately 120 minutes.

8. The suspension temperature was held at 60-70 °C (target 65 °C). The vitamin E TPGS solution prepared in step #7 was slowly added with continuous stirring using Lightnin mixer and mixed for approximately 15-30 min.

9. The suspension was cooled to 23 - 27 °C (target 25 °C). The suspension was visually inspected to ensure that no particles remain.

10. Once the temperature reached 23-27 °C (target 25 °C), BZA was added to the vortex. Once added, the high shear mixer was turned on and mixing was continued until fully dispersed. Excess foaming should be avoided. Time to complete dispersion was recorded (approximately 15-30 minutes). Contents were visually inspected to ensure that the suspension was uniformly dispersed. Any adjustments made to the mixers were recorded.

11. All the mixers were turned off and tank walls and mixer shaft were scraped. The mixers were restarted using previous settings and mixed. Additional water was added if necessary (purified) to achieve theoretical weight, and mixing was continued for an additional 10-15 min. Contents were visually inspected to ensure that the suspension was uniformly dispersed. The high shear mixer was turned off.

12. The mixing was continued with the mixer at slow speed during application, while temperature was maintained at 23-27 °C (target 25 °C). All the mixer speeds were recorded.

Note: All the above steps can be carried out at ambient temperature if suitable equipment is not available. However, the mixing times may have to be extended in order for the excipients to dissolve or suspend. The sucrose palmitate, HPMC and sucrose may be added as a blend at 50-65 °C (target 60 °C) if the mixing vessel is under vacuum. The BZA, ascorbic acid and sucrose may be added as a blend at 23-27 °C (target 25 °C) if the mixing vessel is under vacuum.

The quantitative formula for 1 kg of bazedoxifene acetate filler suspension (20% w/w solids) is shown in Table 8.

Table 8

| INGREDIENT | AMOUNT |
|---|---|
| Sucrose, NF | 104.4 g |
| Hypromellose, USP 2910, 3 cps | 40.0 g |
| Bazedoxifene acetate (@ 88.68% BZ free base) | 45.12* g |
| Sucrose palmitic acid ester | 2.50 g |
| Vitamin E TPGS | 8.00 g |
| Water, USP, purified | 800 g |
| * The potency of bazedoxifene acetate may vary, and the amount in the formula should be adjusted accordingly with a corresponding adjustment in the amount of sucrose to maintain a 20% (w/w) solids content in the suspension. | |

**VI. Formulation of BZA/CE Tablet with Vitamin E TPGS**

Tablets produced according to the above method had the following formulation as shown in Table 9.

Table 9

| | **Amount per unit** | **% wt/wt** |
|---|---|---|
| **Tablet Core** | | |
| Conjugated estrogens inert filled tablet | 210 (1 Tab) mg | 63.64 |
| **Outer Laver (Active Coat)** | | |
| Sucrose, NF | 52.2 mg) | 15.83 |
| Hypromellose, USP 2910, 3 cps | 20.0 mg | 6.06 |
| Bazedoxifene acetate, micronized (@88.68% bazedoxifene free base)^ | 22.56 mg | 6.84 |
| Sucrose palmitic acid ester | 1.25 mg | 0.38 |
| Vitamin E TPGS | 4.00 mg | 1.2 |
| Water, USP purified* | N/A | |
| **Color Coat** | | |
| Opadry Brown (03B96519) | 16.0 mg | 4.85 |
| Water, USP, purified* | N/A | |
| **Clear Coat** | | |
| Opaglos 2 Clear (98Z19173) | 4.00 mg | 1.21 |
| Water, USP, purified* | N/A | |
| **Branding** | | |
| Opacode black ink (WB, NS-78-17821) | 0.20 mg (trace solids) | N/A |
| Water, USP, purified* | N/A | |
| **TOTAL** | 330 mg | 100 |

* Used in the process, but did not appear in the final product.
^ The potency of bazedoxifene may vary; the amount in the formula should be adjusted accordingly with a corresponding adjustment in the amount of sucrose.
Note: The quantities of the ingredients per tablet represent theoretical amounts of coating solids applied. For elegance, the amount of water and coating ingredients used may vary. These may not exceed ± 10% of the theoretical values.

Example 3

Dissolution of Bazedoxifene in BZA/CE Tablets (AA/Tween® Method

The dissolution of bazedoxifene was determined using Apparatus 1 (baskets) at 75 rpm, in 900 mL of 10 mM acetic acid solution with 0.2% polyoxyethylene sorbitan monooleate (Polysorbate 80 or Tween® 80) at 37°C) ± 0.5°C. Samples were analyzed by either online UV (fiber- optic) or off- line UV. For online UV analysis of dissolution samples, the absorbances at 300 nm (wavelength at or near maximum absorbance) and 360 nm (baseline correction wavelength) were determined simultaneously in- situ at the time (s) specified using a UV spectrophotometer; for off- line UV analysis, a filtered sample of dissolution medium was taken at the time (s) specified, and the same absorbances are determined. In both analyses, the amount of bazedoxifene dissolved was determined by subtracting the absorbance at 360 nm from the absorbance at 300 nm and comparing this absorbance to that of a standard solution.

Preparation of Dissolution Medium

1. 20 g of Tween® 80 was dissolved in about 500 mL of USP purified water by stirring while heating until the Tween® 80 was fully dissolved, which can be confirmed by visual inspection.

2. 6 mL of glacial acetic acid and dissolved Tween® 80 solution was transferred to a 10 L container containing 8 L of water.

3. The resulting solution was diluted to 10 L with purified water.

Note: Adjustments may be made in the volume prepared as long as the ratios are kept constant. Proper degassing of dissolution media is necessary for online UV analysis. A solution of 0.02 N HCl may serve as an alternative dissolution medium, instead of AA/Tween®.

Preparation of Standard

Two bazedoxifene acetate reference standard solutions were prepared in separate low actinic volumetric flasks with a final concentration equal to that of the label claim of the tablets being tested fully dissolved in 900 mL dissolution media as follows:

1. The amount of bazedoxifene acetate reference standard shown in the table below was accurately weighed in duplicate (labeled as stock standard A and B) into 50 mL low actinic volumetric flasks and dissolved and diluted to volume with stock standard diluting solution. These were the stock standard preparations A and B.

2. As per the table below, for each stock solution, 7 mL was diluted into a 250 mL low actinic volumetric flask and diluted to volume with dissolution media. These were working standard solutions A and B.

Standard Solution Preparation

| Label Claim (mg) | mg of Reference Standard | Volumetric Flask (mL) | Dilution for Working Standard Solution |
|---|---|---|---|
| 20 | 45 | 50 | 7 mL/250 mL |
| Note: Alternate weights and dilutions may be used, provided that the ratios and final concentration remain unchanged. If this is done, appropriate changes must be made to the calculations. Working standard solutions should be prepared in flasks of at least 250 mL. | | | |

Dissolution Procedure

1. Dissolution was proceeded as directed in the USP (United States Pharmacopeia and National Formulary), using Apparatus 1 for dissolution at 75 rpm using 900 mL of dissolution media.

2. The absorbances of the standard and the sample preparations were measured against the blank solution (dissolution media) over the range of approximately 240 nm to 400 nm and the analytical absorbance was determined at 300 nm and the baseline correction absorbance at 360 nm.

Calculations

1. The absorbance ($A_{T1,T2,T3}$...) of the bazedoxifene sample solutions at time T1, 2, 3... and the absorbance ($A_R$) for the reference standard solution were obtained.
2. The bazedoxifene concentration (Cr) (mg/mL) of the working standard solution was calculated as follows:

$$Cr\ (mg/mL) = (W_r)(S)(V_r2)/(V_r1)(Vr3)$$

where:

$W_r$ = weight of the reference standard, mg
S = use-at value of the reference standard as free base, decimal
$V_r1$ = total volume of stock standard solution, mL
$V_r2$ = pipetted amount of stock standard solution, mL
$V_r3$ = volume of the working standard solution, mL

3. The amount in milligrams ($R_{T1,T2,T3...}$) of bazedoxifene released for each sample at time T1, 2, 3... was calculated

$$R_{T1} = (A_{T1})(C_R)(V)/(A_R)$$

$$R_{T2} = (A_{T2})(C_R)(V)/(A_R)$$

$$R_{T3} = (A_{T3})(C_R)(V)/(A_R)$$

where:

V = volume of media, mL

4. The percent of bazedoxifene released ($\%R_{T1,T2,T3}$...) at time T1, 2, 3... was calculated

$$\% R_{T1} = (R_{T1})(100)/LC$$

$$\% R_{T2} = (R_{T2})(100)/LC$$

$$\% R_{T3} = (R_{T3})(100)/LC$$

where:

LC = label claim of the tablet, mg

Example 4

Dissolution of Tablets Containing Bazedoxifene Acetate, Ascorbic Acid and Hydroxypropyl Methylcellulose

The dissolution of tablets containing bazedoxifene acetate, ascorbic acid and hydroxypropyl methylcellulose were tested according to procedures analogous to those described in Example 3. The testing results are shown in Figures 1-3.

The tablets used in the testing had the following composition as shown in Table 10 and were prepared according procedures analogous to those described in Example 1.

Table 10

| Ingredient | % WT/WT | Input/ DosageUnit Input | Unit |
|---|---|---|---|
| **Core** | | | |
| Conjugated estrogens inert filled tablets | 63.64 | 210 | mg |
| **Outer Layer** | | | |
| Sucrose, NF | 16.42 | 54.2 | mg |
| Hypromellose, USP, 2910, 3 cps | 6.06 | 20.0 | mg |
| Water, USP purified (A) | | N/A | mg |
| Sucrose palmitic acid ester | 0.38 | 1.25 | mg |
| Ascorbic acid, USP | 0.61 | 2.00 | mg |
| Bazedoxifene acetate (@ 88.68% Bazedoxifene free base) (C) | 6.84 | 22.56 | mg |
| **Color Coat (B)** | | | |
| Opadry Brown (03B96519) | 4.85 | 16.00 | mg |
| Water, USP purified (A) | | N/A | mg |
| **Clear Coat (B)** | | | |
| Opaglos 2 Clear (98Z19173) | 1.21 | 4.00 | mg |
| Water, USP purified (A) | | N/A | mg |
| **Total** | 100.00 | 330 | mg |
| *Note:<br>A. Removed during processing<br>B. The quantities of the ingredients per tablet represent theoretical amounts of coating solids applied. For elegance, the amount of water and coating ingredients used may vary. These may not exceed ± 10% of the theoretical values. | | | |

The potency of bazedoxifene acetate may vary, and the amount in the formula must be adjusted accordingly with a corresponding adjustment in the amount of sucrose.

As shown in Figure 2, a substantial retardation in the dissolution rate of bazedoxifene acetate tablets was observed after the tablets were stored over time. For example, the dissolution rate of bazedoxifene acetate from the tablets stored in an open dish for 4 weeks at 40°C and 75% RH decreased from an initial value of about 98% to about 20% at 30 min. when tested with Apparatus 1 as described in the United States Pharmacopoeia (USP29- NF24, page 2673) having a rotation speed of 75 rpm, and a single stage dissolution medium containing acetic acid and Tween® 80 having a pH value of about 4.0 to about 4.5 at about 37°C.

Example 5

Determination of degradants of bazedoxifene in BZA/CE tablets

Degradation of bazedoxifene primarily results in the formation of two major degradation products, N-oxide and cleavage product. Other degradants that may appear on long-term storage are the keto and the aldehyde products. Levels of bazedoxifene degradants were determined as follows. Tablets containing bazedoxifene acetate (BZA) were extracted and diluted with sample solvent (other forms, such as tablets-in-capsule and suspensions, may also be used). A portion of the sample preparation was chromatographed on a reversed phase high-performance liquid chromatography column using gradient elution. The degradants were determined by comparing their peak response in the sample chromatogram with that of the bazedoxifene standard obtained concomitantly.

Reagent and Bulk Solution Preparation

Phosphate Solution - 6.8 g of monobasic potassium and 1.68 g of hexanesulfonic acid sodium salt were dissolved in 2 liter water. The pH of this solution was adjusted to 3.0 with phosphoric acid or NaOH solution.
Sample Solvent - 500 mL of phosphate solution and 500 mL of acetonitrile (ACN) were mixed.
Dilution Solvent - Equal volumes of acetonitrile and water were mixed.
Mobile Phase A - About 400 mg of $NH_4OAc$ was dissolved in 700 mL of water. 150 mL of ACN, 150 mL of MeOH were added and mixed well. Degas if necessary.
Mobile Phase B - About 400 mg of $NH_4OAc$ was dissolved in 200 mL of water. 650 mL of ACN, 150 mL of MeOH were added and mixed well. Degas if necessary.
Note: The volume may be adjusted as long as the ratio among the components used remains constant. The mobile phase solutions are stable for at least 50 days when stored at ambient conditions. Keep the mobile phase solutions tightly capped when not in use.

Control Solution Preparation

1. BZA degradants stock solutions: About 8 mg of cleavage, 4 mg each of keto and N-oxide, and 3 mg aldehyde were weighed into four separate 50 mL flasks and dissolved in the dilution solvent.

2. Stock control solution: About 57 mg of bazedoxifene acetate reference standard was weighed and transferred to a 50 mL volumetric flask. About 30 mL of sample solvent was added. The solution was sonicated for at least ten minutes to dissolve. 2 mL of each degradant stock solution prepared above was pipetted into this flask and diluted to volume with sample solvent. This stock control sample preparation contained bazedoxifene, and each of cleavage, keto, N-oxide, and aldehyde products for peak identification.

3. Ascorbic acid solution: A fresh solution was prepared by weighing about 20 mg ascorbic acid into a 100 mL volumetric flask and dissolved and diluted to volume with sample solvent.

4. Working control solution 1: Equal accurate volumes of the stock control solution and the sample solvent were mixed.

5. Working control solution 2: Equal accurate volumes of the stock control solution to 50 μL ascorbic acid solution were mixed.

Note: Degradants (cleavage, keto, N-oxide, and aldehyde products) are used for peak identification. The accurate weights of these degradants are not necessary. It is suitable to weigh the degradants using an analytical balance.

Signal Verification Solution Preparation

5 mL of the working standard preparation was pipetted into a 100 mL volumetric flask, and diluted to volume with sample solvent. This was the signal-to-noise (S/N) solution with a bazedoxifene concentration of 0.5 μg/mL.

Standard Preparation

Stock Standard

1. About 57 mg (Wr) of bazedoxifene acetate (BZA) reference standard was weighed in duplicate and transferred into two separate 50 mL volumetric flasks. About 30 mL of dilution solvent was added.

2. Both solutions were sonicated for about ten minutes to dissolve, then diluted to volume with the dilution solvent. These were the BZA stock standard and check standard solutions. Each contained about 1 mg/mL of bazedoxifene.

Working Standard

Into two separate 200 mL volumetric flasks, 2.0 mL of each stock standard solution was pipetted, diluted to

volume with sample solvent and mixed well. These were the BZA working standard and check standard solutions.

Note: The volume pipetted (at least 2 mL) and the volume of the standard preparation may be changed, provided that the dilution factor remains constant. Suitable adjustments must be made to the calculations.

Sample Preparation

1. 10 tablets were placed into a glass container of appropriate size (50 - 100% larger than the anticipated volume of the sample solvent use; see table below).

2. The appropriate volume of sample solvent was added to the container and capped tightly. Using a mechanical shaker, the sample was vigorously shaken for approximately 60 - 90 minutes.

Note: Because the extraction process was designed to dissolve BZA from the tablet coating, it was possible that some or all of the tablet cores might not completely disintegrate.

3. A portion of the sample was centrifuged to obtain a clear solution. This was the working sample preparation (1 mg/mL bazedoxifene).

Suggested Volumes for Tablet Sample Preparation

| Tablet Strength mg bazedoxifene | Sample Solvent Volume (Vs) mL |
|---|---|
| 10.0 | 100.0 |
| 20.0 | 200.0 |
| 30.0 | 300.0 |
| 40.0 | 400.0 |

Equipment preparation

1. The detector was set to 220 nm.
2. The column temperature was set at 30 °C.
3. Injection volume was set to 20 $\mu$L.
4. The flow rate was set at 1.0 mL per minute.
5. Gradient Program - Mobile phase B was pumped through the column for 20 minutes and switched to the initial condition (at 30% B) for 20 minutes or until a stable baseline was obtained, then followed the linear gradient program below.

| Time (min) | % A | %B |
|---|---|---|
| 0 | 70 | 30 |
| 1 | 70 | 30 |
| 5 | 65 | 35 |
| 26 | 25 | 75 |
| 26.5 | 70 | 30 |
| 35 | 70 | 30 |

Chromatographic Procedure

1. The sample solvent was injected twice. The first injection was used as column conditioning.

2. Working control solution 1 was injected.

3. Working control solution 2 containing ascorbic acid was injected only if the test sample contained ascorbic acid.

4. The sample solvent was injected again to clean the system.

5. The S/N verification solution was injected; the S/N value should be > 10.

6. The standard preparation was injected. The retention time of bazedoxifene peak should be between 16 and 26 minutes.

7. Two more injections of the standard preparation were performed. The C. V. of all the standard injections (including those after sample injections) should not be more than 5.0%.

8. The check standard preparation was injected. The agreement of BZA in the check standard preparation vs. the standard preparation must be between 95.0-105.0%, according to the following calculation:

$$\% \text{ agreement} = (Ac)(Ws)(100)/(As)(Wc)$$

where:

| | |
|---|---|
| As | = Average peak area response of bazedoxifene from the standard preparation |
| Ws | = Weight of bazedoxifene acetate used in preparing the standard, mg |
| Ac | = Peak area response of bazedoxifene from the check standard preparation |
| Wc | = Weight of bazedoxifene acetate used in preparing the check standard, mg |

9. The sample preparation was injected.

10. The standard preparation was injected after the last sample preparation injection. In extended runs of sample injections, the standard preparation was re-injected after every 5-10 sample injections.

Calculations

Standard Concentrations

The concentration of bazedoxifene (BZ) was calculated in each standard preparation (Cr) from the following equation:

$$Cr\ (mg/mL) = (Wr)(S)/(Vr)(D)$$

where:

| | |
|---|---|
| Wr | = weight of bazedoxifene acetate reference standard, mg |
| S | = use-at value of the bazedoxifene free base, decimal |
| Vr | = volume of the stock standard solution, mL |
| D | = additional dilution factor for standard preparations |

Degradant Calculation

1. The area of peaks in the sample chromatogram that did not have the same retention times as bazedoxifene were determined. Peaks that had the same retention times and areas as peaks in the blank chromatogram were ignored. Peaks eluted in the first 4 minutes (which are from the conjugated estrogens core) were ignored.

2. Percent degradant in tablets/tablets-in-capsule were calculated from the equation:

$$\%degradant = (A_{spl})(Cr)(Vs)(Ds)(RF)(100)/(A_{std})(LC)(N)$$

where:

| | |
|---|---|
| $A_{spl}$ | = area of degradant peak from sample chromatogram |
| Cr | = standard concentration, mg/mL |
| $A_{std}$ | = average area of BZ from the standard preparation chromatograms |
| Vs | = volume of sample preparation, mL |
| Ds | = sample dilution factor |
| RF | = response factor for degradants (relative to BZ) |
| 100 | = conversion factor, % |
| LC | = label claim of BZ, mg/tablet |
| N | = number of tablets |

3. Response factors for various known degradants:

Cleavage product = 0.813

Keto = 1.116

N-oxide = 1.013

Aldehyde = 1.247

Example 6

Bioavailability Study of Tablets Containing Bazedoxifene Acetate and Ascorbic Acid or Vitamin E

Bioavailability of bazedoxifene in BZA/CE tablets was studied in approximately 24 healthy postmenopausal women, to allow completion in at least 22 subjects. Test article and reference therapy were as follows:

- Treatment A (test article): vitamin E-containing formulation of BZA/CE 20 mg/0.625 mg combination tablet (Form A); and
- Treatment B (reference therapy): Ascorbic acid-containing formulation of BZA/CE 20 mg/0.625 mg combination tablet.

In each period, subjects received either a single dose of test article, Treatment A, or reference therapy, Treatment B, according to 1 of 2 randomization sequences. Subjects received each single oral dose with 240 mL of room-temperature water after an overnight fast of at least 10 hours on day 1 at approximately 0800. Blood samples were drawn at specified times for 24 hours, and plasma was separated and assayed for BZA and conjugated estrogens (estrone only). As shown in Figure 16 and Table 2, the bioavailability of BZA in a BZA/CE formulation with 1 mg/tablet vitamin E was higher than that of a formulation with 2 mg/tablet ascorbic acid ("Form A" in Figure 16), both in terms of $C_{max}$ (highest concentration reached) and AUC (area under the curve; total exposure). Specifically, $C_{max}$ and AUC increased about 43% and about 12% respectively when ascorbic acid (1 mg/tablet) in the outer layer containing BZA was replaced with vitamin E (1 mg/tablet).

## Claims

1. A method of enhancing dissolution stability of bazedoxifene in a pharmaceutical composition comprising bazedoxifene, or a pharmaceutically acceptable salt thereof, the method comprising formulating the pharmaceutical composition to comprise at least one of vitamin E and vitamin E TPGS, wherein the pharmaceutical composition is substantially free of ascorbic acid.

2. A pharmaceutical composition comprising bazedoxifene, or a pharmaceutically acceptable salt thereof, a filler, a

binder, a wetting agent, and at least one of vitamin E and vitamin E TPGS, wherein the pharmaceutical composition is substantially free of ascorbic acid, wherein the dissolution stability of bazedoxifene in the pharmaceutical composition is enhanced compared to the dissolution stability of bazedoxifene in a pharmaceutical composition comprising bazedoxifene and ascorbic acid.

**3.** The pharmaceutical composition of claim 2, wherein the pharmaceutical composition comprises from about 10% to about 40% by weight as bazedoxifene.

**4.** The pharmaceutical composition of claim 2, wherein
the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosillicates, calcium phosphate, and metal carbonate;
the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, copovidone, xanthan gum, and guar gum; and
the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides.

**5.** The pharmaceutical composition of claim 4, wherein the filler is sucrose.

**6.** The pharmaceutical composition of claim 4, wherein the binder is hydroxypropyl methylcellulose.

**7.** The pharmaceutical composition of claim 4, wherein the wetting agent is sucrose palmitic acid ester.

**8.** The pharmaceutical composition of claim 4, wherein the filler is sucrose, the binder is hydroxypropyl methylcellulose, and the wetting agent is sucrose palmitic acid ester.

**9.** A pharmaceutical composition, comprising:

a core comprising conjugated estrogens; and
at least one coating comprising bazedoxifene, or a pharmaceutically acceptable salt thereof, a filler, a binder, a wetting agent, and at least one of vitamin E and vitamin E TPGS,
wherein the at least one coating is substantially free of ascorbic acid, and
wherein the dissolution stability of bazedoxifene in the pharmaceutical composition is enhanced compared to the dissolution stability of bazedoxifene in a pharmaceutical composition comprising bazedoxifene and ascorbic acid.

**10.** The pharmaceutical composition of claim 9, wherein the pharmaceutical composition comprises from about 3% to about 10% by weight of the total composition as bazedoxifene acetate, from about 5% to about 30% by weight of the total composition as filler, from about 3% to about 10% by weight of the total composition as binder, from about 0.01 % to about 2% by weight of the total composition as wetting agent, and from about 0.01 % to about 2% by weight of the of the total composition as at least one of vitamin E and vitamin E TPGS.

**11.** The pharmaceutical composition of claim 10, wherein the pharmaceutical composition comprises about 0.1 % or greater by weight of the at least one of vitamin E and vitamin E TPGS.

**12.** The pharmaceutical composition of claim 9, wherein:

the filler comprises at least one of lactose, lactose monohydrate, mannitol, sucrose, maltodextrin, dextrin, maltitol, sorbitol, xylitol, powdered cellulose, cellulose gum, microcrystalline cellulose, carboxymethyl cellulose, carboxyethyl cellulose, hydroxyethyl cellulose, starch, sodium starch glycolates, metal aluminosillicates, calcium phosphate, and metal carbonate;
the binder comprises at least one of hydroxypropyl methylcellulose, carboxymethyl cellulose, carboxyethyl cellulose, hydroxypropyl cellulose, hydroxyethyl cellulose, microcrystalline celluloses, starches, polyvinyl pyrrolidine, polyethylene oxide, polyvinyl pyrrolidone, copovidone, xanthan gum, and guar gum; and the wetting agent comprises at least one of sucrose palmitic acid ester, polyethylene glycol-polypropylene glycol copolymer, metal alkyl sulfate, sodium lauryl sulfate, polyoxyethylene sorbitan fatty acid ester, polyoxyethylene alkyl ether, polyethylene glycol, polyoxyethylene castor oil derivatives, docusate sodium, quaternary ammonium amine compounds, sugar esters of fatty acids, polyethoxylated fatty acid esters, glycerides of fatty acids, and polyglycolized glycerides.

**13.** The pharmaceutical composition of claim 12, wherein the filler is sucrose.

**14.** The pharmaceutical composition of claim 12, wherein the filler is sucrose, the binder is hydroxypropyl methylcellulose, and the wetting agent is sucrose palmitic acid ester.

**15.** A pharmaceutical composition comprising:

a core tablet comprising at least one conjugated estrogen, said core tablet forming from about 45% to about 80% by weight of the total composition; and
an outer layer substantially free of ascorbic acid, comprising:

bazedoxifene acetate from about 4% to about 8% by weight of the total composition;
sucrose from about 10% to about 20% by weight of the total composition;
hydroxypropyl methylcellulose from about 4% to about 8% by weight of the total composition;
sucrose palmitic acid ester from about 0.2% to about 0.6% by weight of the total composition; and
at least one of vitamin E and vitamin E TPGS from about 0.1% to about 2% to by weight of the total composition,
provided that the total % of the composition by weight is 100%,
wherein the dissolution stability of bazedoxifene acetate in the pharmaceutical composition is enhanced compared to the dissolution stability of bazedoxifene acetate in a pharmaceutical composition comprising bazedoxifene acetate and ascorbic acid.

## Patentansprüche

**1.** Verfahren zur Erhöhung der Auflösungsstabilität von Bazedoxifen in einer pharmazeutischen Zusammensetzung, die Bazedoxifen oder ein pharmazeutisch annehmbares Salz davon umfasst, wobei das Verfahren Formulieren der pharmazeutischen Zusammensetzung, so dass sie wenigstens eins von Vitamin E und Vitamin E TPGS umfasst, umfasst, wobei die pharmazeutische Zusammensetzung im Wesentlichen frei von Ascorbinsäure ist.

**2.** Pharmazeutische Zusammensetzung, die Bazedoxifen oder ein pharmazeutisch annehmbares Salz davon, einen Füllstoff, ein Bindemittel, ein Benetzungsmittel und wenigstens eins von Vitamin E und Vitamin E TPGS umfasst, wobei die pharmazeutische Zusammensetzung im Wesentlichen frei von Ascorbinsäure ist, wobei die Auflösungsstabilität von Bazedoxifen in der pharmazeutischen Zusammensetzung im Vergleich zu der Auflösungsstabilität von Bazedoxifen in einer pharmazeutischen Zusammensetzung, die Bazedoxifen und Ascorbinsäure umfasst, erhöht ist.

**3.** Pharmazeutische Zusammensetzung gemäß Anspruch 2, wobei die pharmazeutische Zusammensetzung etwa 10 Gew.-% bis etwa 40 Gew.-% Bazedoxifen umfasst.

**4.** Pharmazeutische Zusammensetzung gemäß Anspruch 2, wobei
der Füllstoff wenigstens einen von Lactose, Lactosemonohydrat, Mannit, Saccharose, Maltodextrin, Dextrin, Maltit, Sorbit, Xylit, pulverförmiger Cellulose, Cellulosegummi, mikrokristalliner Cellulose, Carboxymethylcellulose, Carboxyethylcellulose, Hydroxyethylcellulose, Stärke, Natriumstärkeglycolaten, Metallaluminiumsilikaten, Calciumphosphat und Metallcarbonat umfasst;
das Bindemittel wenigstens eins von Hydroxypropylmethylcellulose, Carboxymethylcellulose, Carboxyethylcellulose, Hydroxypropylcellulose, Hydroxyethylcellulose, mikrokristallinen Cellulosen, Stärken, Polyvinylpyrrolidin, Polyethylenoxid, Polyvinylpyrrolidon, Crospovidon, Xanthangummi und Guargummi umfasst und
das Benetzungsmittel wenigstens eins von Saccharosepalmitinsäureester, Polyethylenglycol- Polypropylenglycol-Copolymer, Metallalkylsulfat, Natriumlaurylsulfat, Polyoxyethylensorbitanfettsäureester, Polyoxyethylenalkylether, Polyethylenglycol, Polyoxyethylen- Rizinusöl- Derivaten, Docusatnatrium, quaternären Ammoniumamin- Verbindungen, Zukkerestern von Fettsäuren, polyethoxylierten Fettsäureestern, Glyceriden von Fettsäuren und polyglycolysierten Glyceriden umfasst.

**5.** Pharmazeutische Zusammensetzung gemäß Anspruch 4, wobei der Füllstoff Saccharose ist.

**6.** Pharmazeutische Zusammensetzung gemäß Anspruch 4, wobei das Bindemittel Hydroxypropylmethylcellulose ist.

**7.** Pharmazeutische Zusammensetzung gemäß Anspruch 4, wobei das Benetzungsmittel Saccharosepalmitinsäureester ist.

**8.** Pharmazeutische Zusammensetzung gemäß Anspruch 4, wobei der Füllstoff Saccharose ist, das Bindemittel Hydroxypropylmethylcellulose ist und das Benetzungsmittel Saccharosepalmitinsäureester ist.

**9.** Pharmazeutische Zusammensetzung, umfassend:

einen Kern, der konjugierte Östrogene umfasst, und
wenigstens eine Beschichtung, die Bazedoxifen oder ein pharmazeutisch annehmbares Salz davon, einen Füllstoff, ein Bindemittel, ein Benetzungsmittel und wenigstens eins von Vitamin E und Vitamin E TPGS umfasst, wobei die wenigstens eine Beschichtung im Wesentlichen frei von Ascorbinsäure ist und
wobei die Auflösungsstabilität von Bazedoxifen in der pharmazeutischen Zusammensetzung im Vergleich zu der Auflösungsstabilität von Bazedoxifen in einer pharmazeutischen Zusammensetzung, die Bazedoxifen und Ascorbinsäure umfasst, erhöht ist.

**10.** Pharmazeutische Zusammensetzung gemäß Anspruch 9, wobei die pharmazeutische Zusammensetzung umfasst: etwa 3 Gew.-% bis etwa 10 Gew.-% der gesamten Zusammensetzung Bazedoxifenacetat, etwa 5 Gew.-% bis etwa 30 Gew.-% der gesamten Zusammensetzung Füllstoff, etwa 3 Gew.-% bis etwa 10 Gew.-% der gesamten Zusammensetzung Bindemittel, etwa 0,01 Gew.-% bis etwa 2 Gew.-% der gesamten Zusammensetzung Benetzungsmittel und etwa 0,01 Gew.-% bis etwa 2 Gew.-% der gesamten Zusammensetzung wenigstens eins von Vitamin E und Vitamin E TPGS.

**11.** Pharmazeutische Zusammensetzung gemäß Anspruch 10, wobei die pharmazeutische Zusammensetzung etwa 0,1 Gew.-% oder mehr des wenigstens einen von Vitamin E und Vitamin E TPGS umfasst.

**12.** Pharmazeutische Zusammensetzung gemäß Anspruch 9, wobei:

der Füllstoff wenigstens einen von Lactose,
Lactosemonohydrat, Mannit, Saccharose, Maltodextrin, Dextrin, Maltit, Sorbit, Xylit, pulverförmiger Cellulose, Cellulosegummi, mikrokristalliner Cellulose, Carboxymethylcellulose, Carboxyethylcellulose, Hydroxyethylcellulose, Stärke, Natriumstärkeglycolaten, Metallaluminosilikaten, Calciumphosphat und Metallcarbonat umfasst;
das Bindemittel wenigstens eins von Hydroxypropylmethylcellulose, Carboxymethylcellulose, Carboxyethylcellulose, Hydroxypropylcellulose, Hydroxyethylcellulose, mikrokristallinen Cellulosen, Stärken, Polyvinylpyrrolidin, Polyethylenoxid, Polyvinylpyrrolidon, Crospovidon, Xanthangummi und Guargummi umfasst und
das Benetzungsmittel wenigstens eins von Saccharosepalmitinsäureester, Polyethylenglycol- Polypropylenglycol- Copolymer, Metallalkylsulfat, Natriumlaurylsulfat, Polyoxyethylensorbitanfettsäureester, Polyoxyethylenalkylether, Polyethylenglycol, Polyoxyethylen- Rizinusöl- Derivaten, Docusatnatrium, quaternären Ammoniumamin- Verbindungen, Zuckerestern von Fettsäuren, polyethoxylierten Fettsäureestern, Glyceriden von Fettsäuren und polyglycolysierten Glyceriden umfasst

**13.** Pharmazeutische Zusammensetzung gemäß Anspruch 12, wobei der Füllstoff Saccharose ist.

**14.** Pharmazeutische Zusammensetzung gemäß Anspruch 12, wobei der Füllstoff Saccharose ist, das Bindemittel Hydroxypropylmethylcellulose ist und das Benetzungsmittel Saccharosepalmitinsäureester ist.

**15.** Pharmazeutische Zusammensetzung, umfassend:

eine Kerntablette, die wenigstens ein konjugiertes Östrogen umfasst, wobei die Kerntablette etwa 45 Gew.-% bis etwa 80 Gew.-% der gesamten Zusammensetzung bildet, und
eine äußere Schicht, die im Wesentlichen frei von Ascorbinsäure ist, umfassend:

Bazedoxifenacetat mit etwa 4 Gew.-% bis etwa 8 Gew.-% der gesamten Zusammensetzung;
Saccharose mit etwa 10 Gew.-% bis etwa 20 Gew.-% der gesamten Zusammensetzung;
Hydroxypropylmethylcellulose mit etwa 4 Gew.-% bis etwa 8 Gew.-% der gesamten Zusammensetzung;
Saccharosepalmitinsäureester mit etwa 0,2 Gew.-% bis etwa 0,6 Gew.-% der gesamten Zusammensetzung und
wenigstens eins von Vitamin E und Vitamin E TPGS mit etwa 0,1 Gew.-% bis etwa 2 Gew.-% der gesamten Zusammensetzung, mit der Maßgabe, dass die Gesamt-Gewichts-% der Zusammensetzung 100% sind, wobei die Auflösungsstabilität von Bazedoxifenacetat in der pharmazeutischen Zusammensetzung im Vergleich zu der Auflösungsstabilität von Bazedoxifenacetat in einer pharmazeutischen Zusammensetzung, die Bazedoxifenacetat und Ascorbinsäure umfasst, erhöht ist.

**Revendications**

**1.** Procédé d'augmentation de la stabilité de dissolution de bazédoxifène dans une composition pharmaceutique comprenant du bazédoxifène, ou un sel pharmaceutiquement acceptable de celui-ci, le procédé comprenant la formulation de la composition pharmaceutique de façon à ce qu'elle comprenne au moins un composé parmi la vitamine E et la vitamine E TPGS, dans lequel la composition pharmaceutique est sensiblement dépourvue d'acide ascorbique.

**2.** Composition pharmaceutique comprenant du bazédoxifène, ou un sel pharmaceutiquement acceptable de celui-ci, une charge, un liant, un agent mouillant et au moins un composé parmi la vitamine E et la vitamine E TPGS, dans laquelle la composition pharmaceutique est sensiblement dépourvue d'acide ascorbique, dans laquelle la stabilité de dissolution du bazédoxifène dans la composition pharmaceutique est augmentée par rapport à la stabilité de dissolution du bazédoxifène dans une composition pharmaceutique comprenant du bazédoxifène et de l'acide ascorbique.

**3.** Composition pharmaceutique selon la revendication 2, dans laquelle la composition pharmaceutique comprend d'environ 10 % à environ 40 % en poids de bazédoxifène.

**4.** Composition pharmaceutique selon la revendication 2, dans laquelle :

la charge comprend au moins un composé parmi le lactose, le lactose monohydraté, le mannitol, le saccharose, la maltodextrine, la dextrine, le maltitol, le sorbitol, le xylitol, la cellulose pulvérulente, la gomme de cellulose, la cellulose microcristalline, la carboxyméthyl-cellulose, la carboxyéthyl-cellulose, l'hydroxyéthyl-cellulose, l'amidon, les glycolates d'amidon sodiques, les aluminosilicates métalliques, le phosphate de calcium et le carbonate métallique ;
le liant comprend au moins un composé parmi l'hydroxypropylméthyl-cellulose, la carboxyméthyl-cellulose, la carboxyéthyl-cellulose, l'hydroxypropylcellulose, l'hydroxyéthyl-cellulose, les celluloses microcristallines, les amidons, la polyvinylpyrrolidine, le poly(oxyde d'éthylène), la polyvinylpyrrolidone, la copovidone, la gomme xanthane et la gomme guar ; et
l'agent mouillant comprend au moins un composé parmi l'ester d'acide palmitique de saccharose, un copolymère de polyéthylène glycol - polypropylène glycol, un alkylsulfate métallique, le laurylsulfate de sodium, l'ester d'acide gras et de sorbitan polyéthoxylé, un éther alkylique de polyoxyéthylène, le polyéthylène glycol, les dérivés d'huile de ricin polyéthoxylés, le docusate de sodium, les composés d'amine d'ammonium quaternaire, les esters de sucre d'acides gras, les esters d'acides gras polyéthoxylés, les glycérides d'acides gras et les glycérides polyglycolés.

**5.** Composition pharmaceutique selon la revendication 4, dans laquelle la charge est le saccharose.

**6.** Composition pharmaceutique selon la revendication 4, dans laquelle le liant est l'hydroxypropylméthyl-cellulose.

**7.** Composition pharmaceutique selon la revendication 4, dans laquelle l'agent mouillant est l'ester d'acide palmitique de saccharose.

**8.** Composition pharmaceutique selon la revendication 4, dans laquelle la charge est le saccharose, le liant est l'hydroxypropylméthyl-cellulose et l'agent mouillant est l'ester d'acide palmitique de saccharose.

**9.** Composition pharmaceutique, comprenant :

un noyau comprenant des estrogènes conjugués ; et
au moins un revêtement comprenant du bazédoxifène, ou un sel pharmaceutiquement acceptable de celui-ci, une charge, un liant, un agent mouillant et au moins un composé parmi la vitamine E et la vitamine E TPGS,
dans laquelle l'au moins un revêtement est sensiblement dépourvu d'acide ascorbique, et
dans laquelle la stabilité de dissolution du bazédoxifène dans la composition pharmaceutique est augmentée par rapport à la stabilité de dissolution du bazédoxifène dans une composition pharmaceutique comprenant du bazédoxifène et de l'acide ascorbique.

**10.** Composition pharmaceutique selon la revendication 9, dans laquelle la composition pharmaceutique comprend d'environ 3 % à environ 10 % en poids de la composition totale d'acétate de bazédoxifène, d'environ 5 % à environ 30 % en poids de la composition totale d'une charge, d'environ 3 % à environ 10 % en poids de la composition totale d'un liant, d'environ 0,01 % à environ 2 % en poids de la composition totale d'un agent mouillant et d'environ 0,01 % à environ 2 % en poids de la composition totale d'au moins un composé parmi la vitamine E et la vitamine E TPGS.

**11.** Composition pharmaceutique selon la revendication 10, dans laquelle la composition pharmaceutique comprend environ 0,1 % en poids ou plus d'au moins un composé parmi la vitamine E et la vitamine E TPGS.

**12.** Composition pharmaceutique selon la revendication 9, dans laquelle :

la charge comprend au moins un composé parmi le lactose, le lactose monohydraté, le mannitol, le saccharose, la maltodextrine, la dextrine, le maltitol, le sorbitol, le xylitol, la cellulose pulvérulente, la gomme de cellulose, la cellulose microcristalline, la carboxyméthyl-cellulose, la carboxyéthyl-cellulose, l'hydroxyéthyl-cellulose, l'amidon, les glycolates d'amidon sodiques, les aluminosilicates métalliques, le phosphate de calcium et le carbonate métallique ;
le liant comprend au moins un composé parmi l'hydroxypropylméthyl-cellulose, la carboxyméthyl-cellulose, la carboxyéthyl-cellulose, l'hydroxypropylcellulose, l'hydroxyéthyl-cellulose, les celluloses microcristallines, les amidons, la polyvinylpyrrolidine, le poly(oxyde d'éthylène), la polyvinylpyrrolidone, la copovidone, la gomme xanthane et la gomme guar ; et
l'agent mouillant comprend au moins un composé parmi l'ester d'acide palmitique de saccharose, un copolymère de polyéthylène glycol - polypropylène glycol, un alkylsulfate métallique, le laurylsulfate de sodium, l'ester d'acide gras et de sorbitan polyéthoxylé, un éther alkylique de polyoxyéthylène, le polyéthylène glycol, les dérivés d'huile de ricin polyéthoxylés, le décusate de sodium, les composés d'amine d'ammonium quaternaire, les esters de sucre d'acides gras, les esters d'acides gras polyéthoxylés, les glycérides d'acides gras et les glycérides polyglycolés.

**13.** Composition pharmaceutique selon la revendication 12, dans laquelle la charge est le saccharose.

**14.** Composition pharmaceutique selon la revendication 12, dans laquelle la charge est le saccharose, le liant est l'hydroxypropylméthyl-cellulose et l'agent mouillant est l'ester d'acide palmitique de saccharose.

**15.** Composition pharmaceutique, comprenant :

un comprimé à noyau comprenant au moins un estrogène conjugué, ledit comprimé à noyau constituant d'environ 45 % à environ 80 % en poids de la composition totale ; et
une couche extérieure sensiblement dépourvue d'acide ascorbique, comprenant :

de l'acétate de bazédoxifène à raison d'environ 4 % à environ 8 % en poids de la composition totale ;
du saccharose à raison d'environ 10 % à environ 20 % en poids de la composition totale ;
de l'hydroxypropylméthyl-cellulose à raison d'environ 4 % à environ 8 % en poids de la composition totale ;
de l'ester d'acide palmitique de saccharose à raison d'environ 0,2 % à environ 0,6 % en poids de la composition totale ; et
au moins un composé parmi la vitamine E et la vitamine E TPGS à raison d'environ 0,1 % à environ 2 % en poids de la composition totale,
à condition que le % total en poids de la composition soit de 100 %,
dans laquelle la stabilité de dissolution du bazédoxifène dans la composition pharmaceutique est augmentée

par rapport à la stabilité de dissolution du bazédoxifène dans une composition pharmaceutique comprenant du bazédoxifène et de l'acide ascorbique.

FIGURE 1

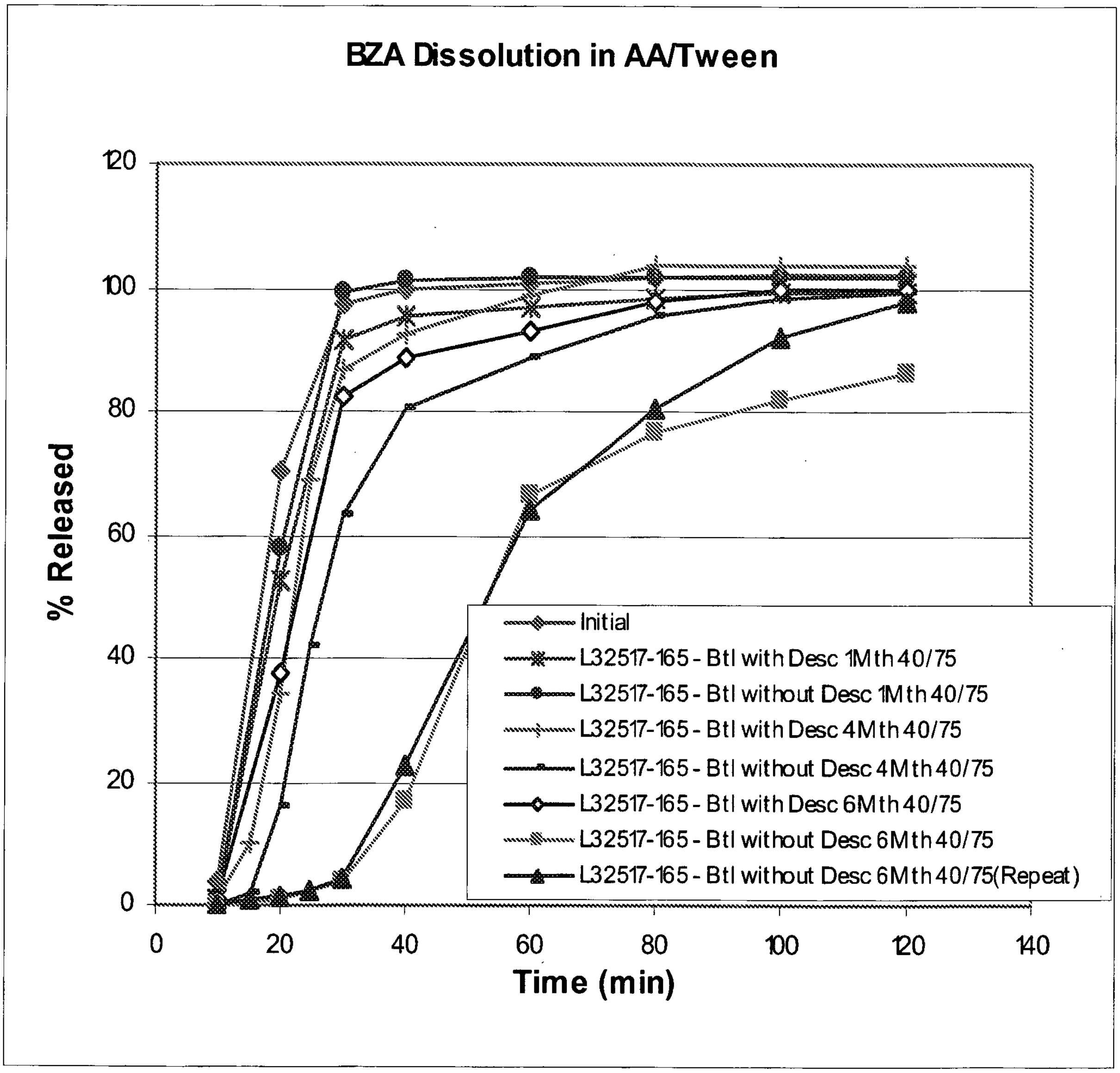
BZA Dissolution in AA/Tween
% Released
Time (min)
0
20
40
60
80
100
120
140
Initial
L32517-165 - Btl with Desc 1Mth 40/75
L32517-165 - Btl without Desc 1Mth 40/75
L32517-165 - Btl with Desc 4Mth 40/75
L32517-165 - Btl without Desc 4Mth 40/75
L32517-165 - Btl with Desc 6Mth 40/75
L32517-165 - Btl without Desc 6Mth 40/75
L32517-165 - Btl without Desc 6Mth 40/75(Repeat)

FIGURE 2

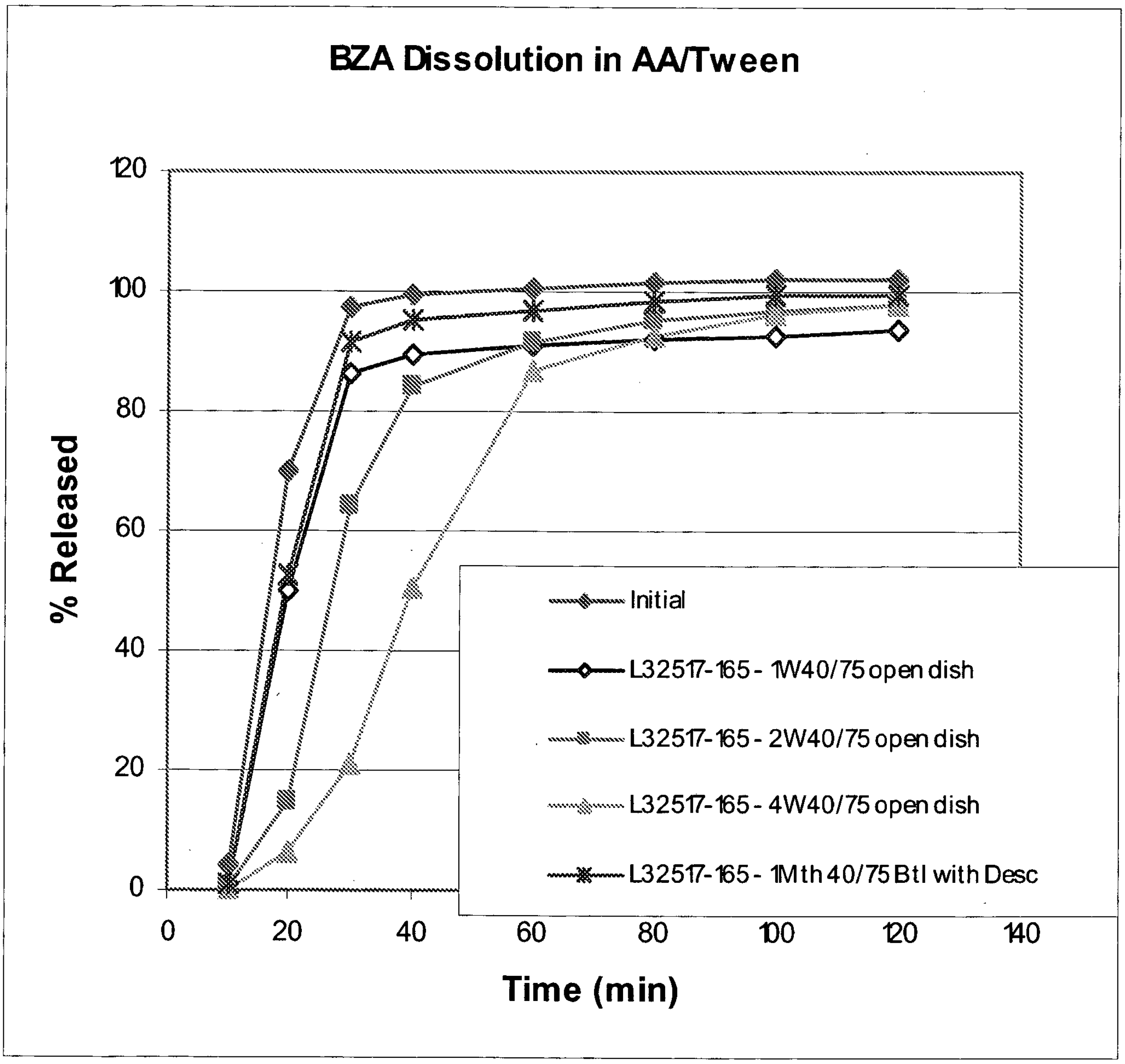
BZA Dissolution in AA/Tween
% Released
Time (min)
0
20
40
60
80
100
120
140
Initial
L32517-165 - 1W40/75 open dish
L32517-165 - 2W40/75 open dish
L32517-165 - 4W40/75 open dish
L32517-165 - 1Mth 40/75 Btl with Desc

FIGURE 3

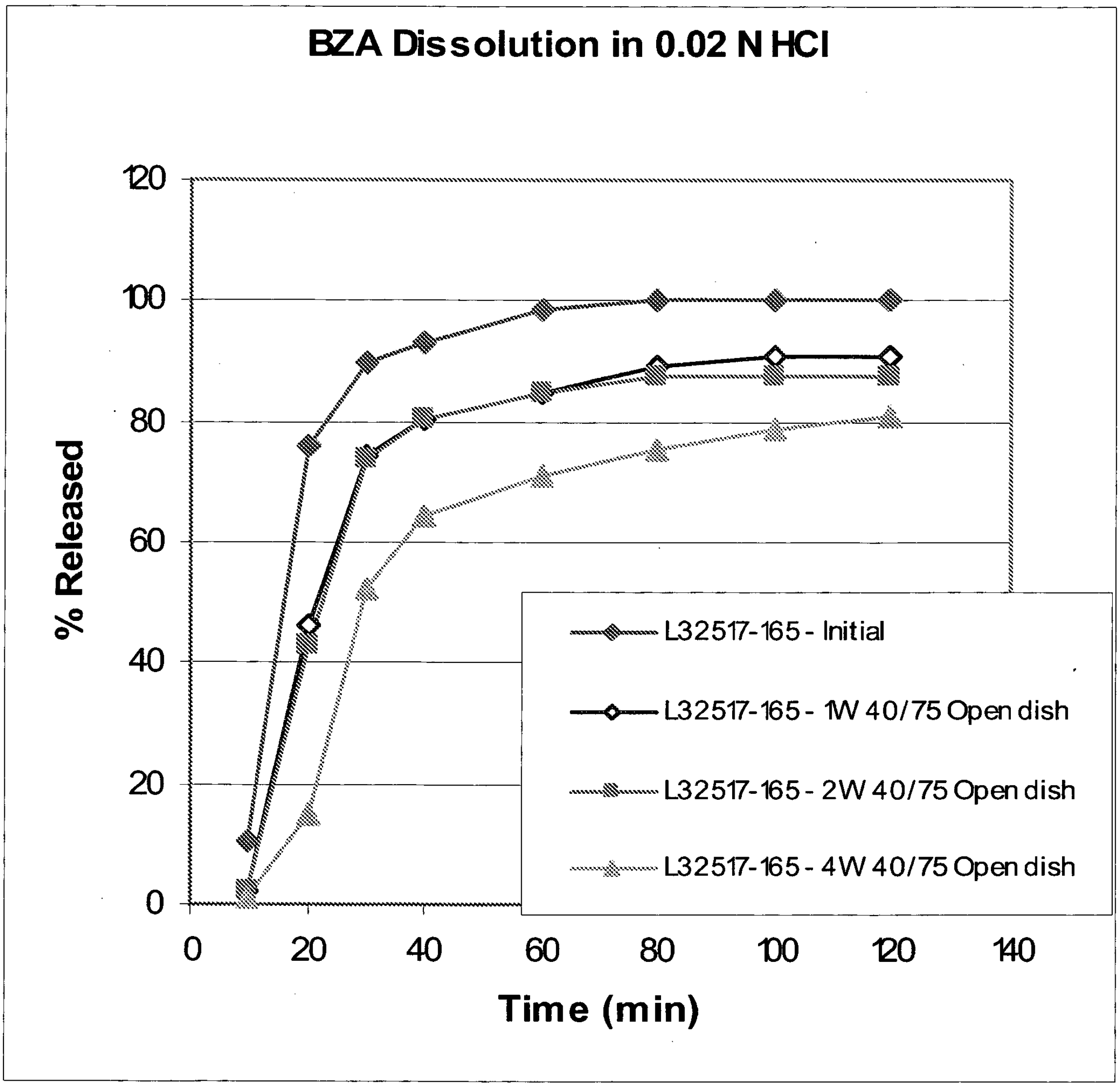
BZA Dissolution in 0.02 N HCl
% Released
120
100
80
60
40
20
0
0
20
40
60
80
100
120
140
Time (min)
L32517-165 - Initial
L32517-165 - 1W 40/75 Open dish
L32517-165 - 2W 40/75 Open dish
L32517-165 - 4W 40/75 Open dish

FIGURE 4

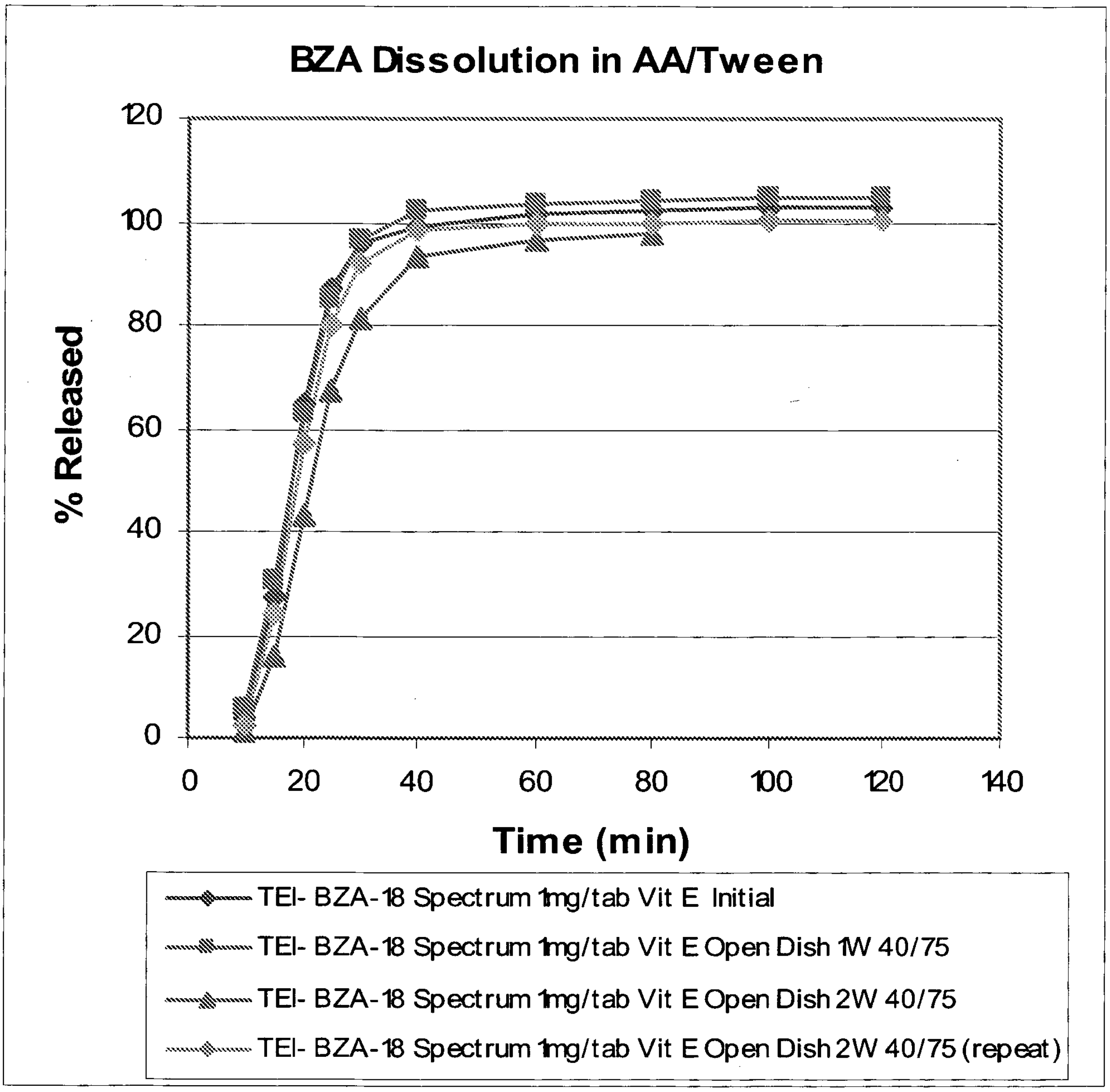
BZA Dissolution in AA/Tween
120
100
80
60
40
20
0
% Released
0
20
40
60
80
100
120
140
Time (min)
TEI- BZA-18 Spectrum 1mg/tab Vit E Initial
TEI- BZA-18 Spectrum 1mg/tab Vit E Open Dish 1W 40/75
TEI- BZA-18 Spectrum 1mg/tab Vit E Open Dish 2W 40/75
TEI- BZA-18 Spectrum 1mg/tab Vit E Open Dish 2W 40/75 (repeat)

FIGURE 5

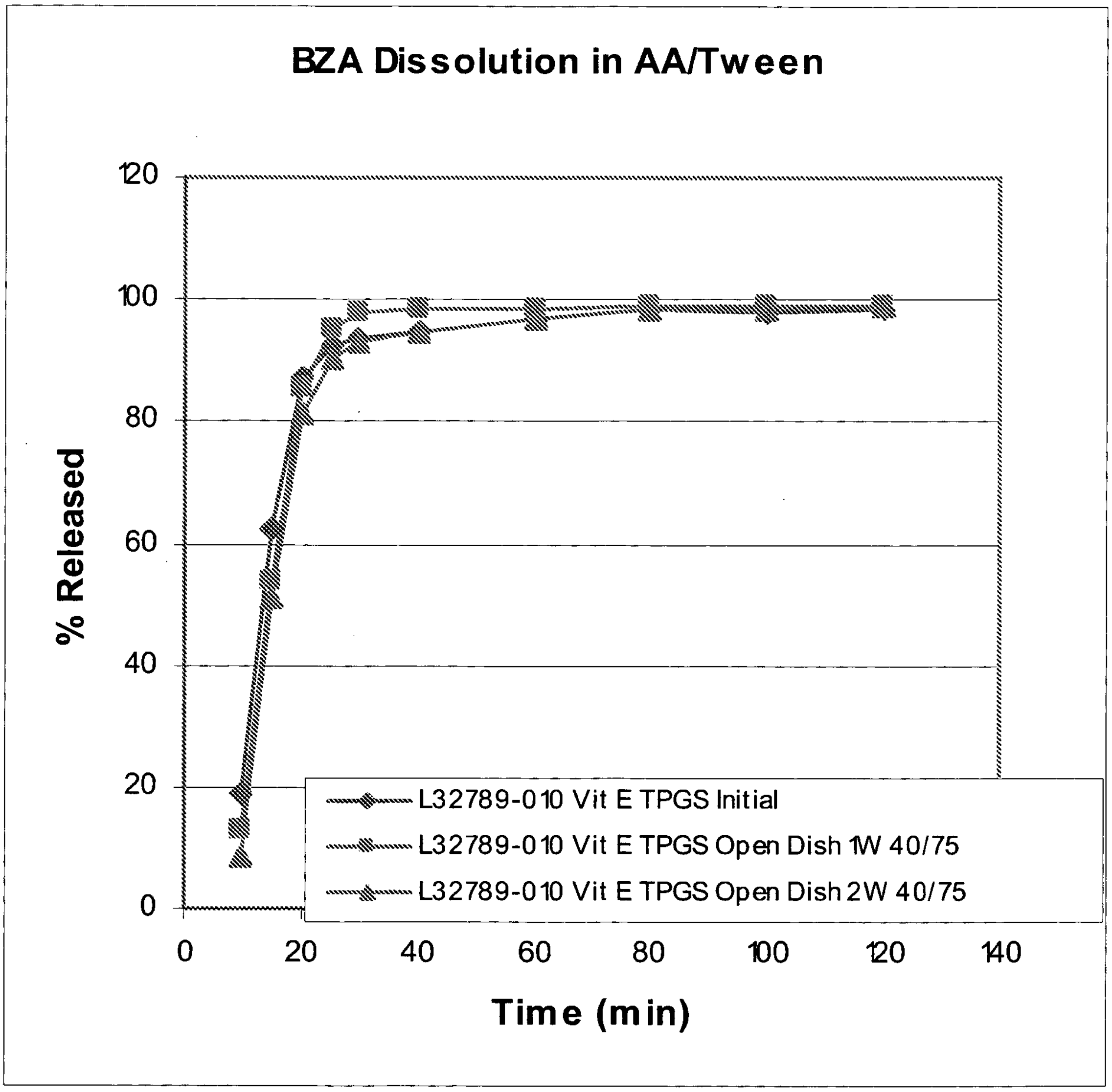
BZA Dissolution in AA/Tween
120
100
80
60
40
20
0
% Released
0
20
40
60
80
100
120
140
Time (min)
L32789-010 Vit E TPGS Initial
L32789-010 Vit E TPGS Open Dish 1W 40/75
L32789-010 Vit E TPGS Open Dish 2W 40/75

FIGURE 6

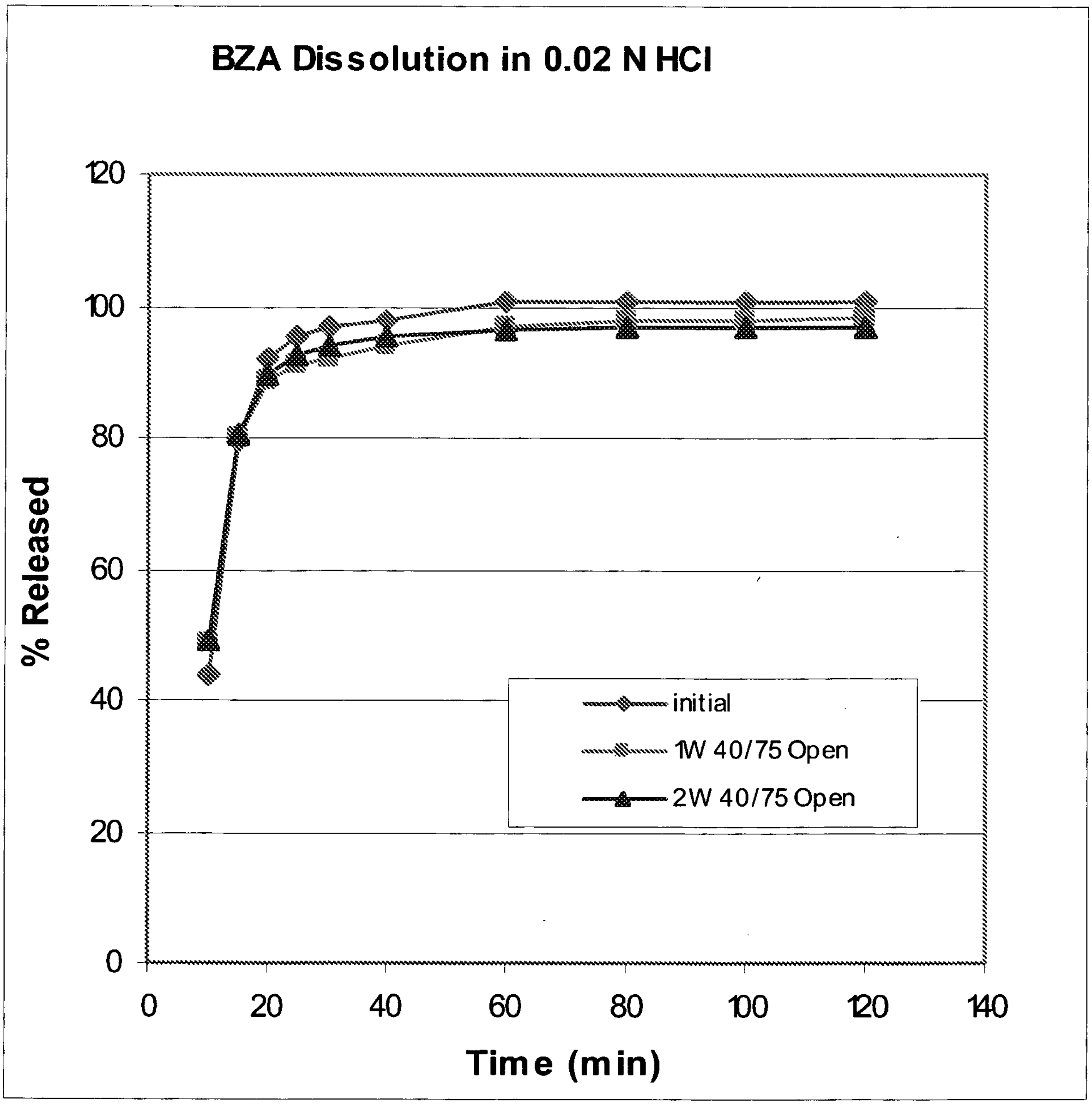
BZA Dissolution in 0.02 N HCl
% Released
Time (min)
0
20
40
60
80
100
120
140
initial
1W 40/75 Open
2W 40/75 Open

FIGURE 7

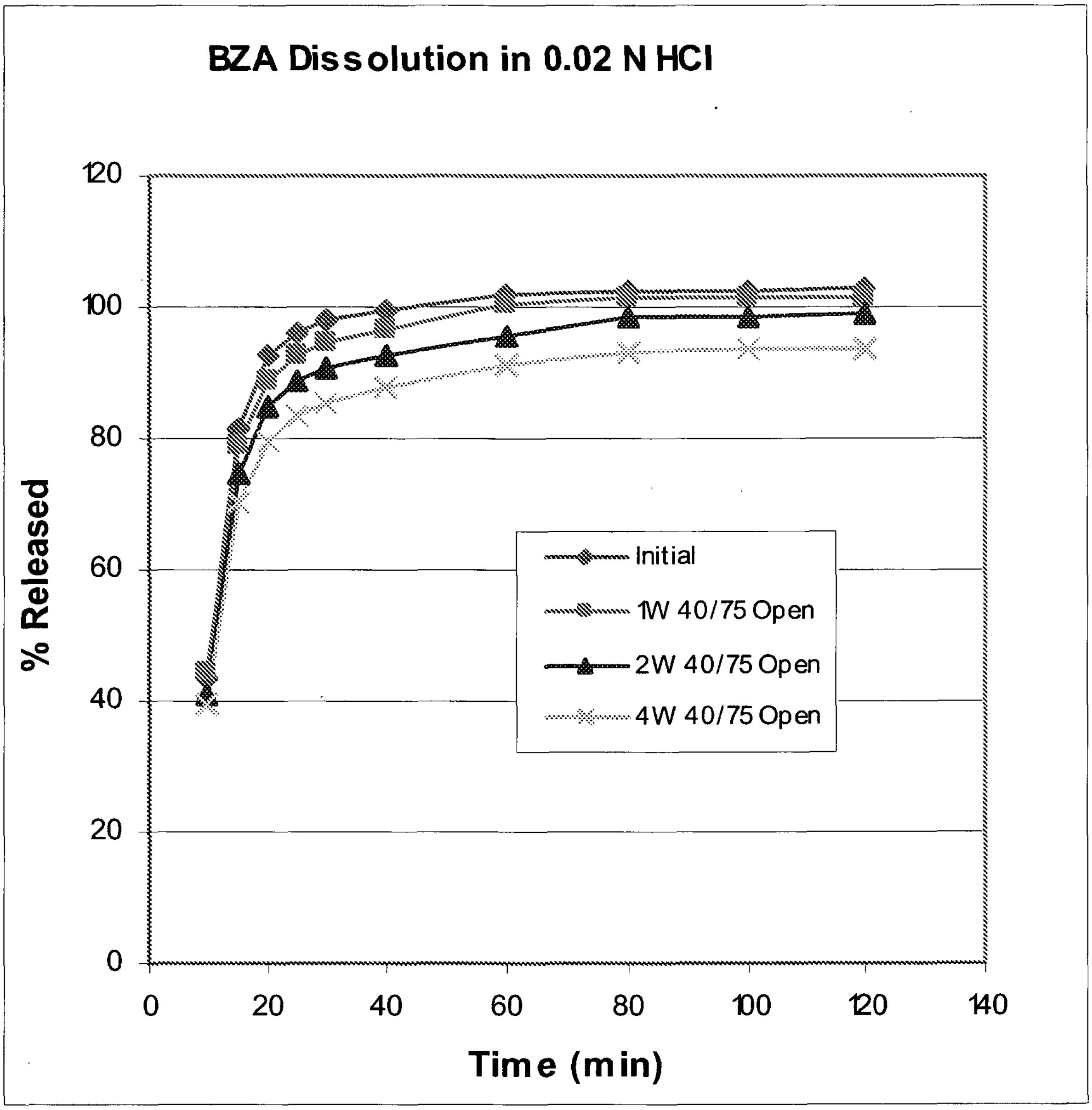
BZA Dissolution in 0.02 N HCl
% Released
Time (min)
0
20
40
60
80
100
120
140
Initial
1W 40/75 Open
2W 40/75 Open
4W 40/75 Open

FIGURE 8

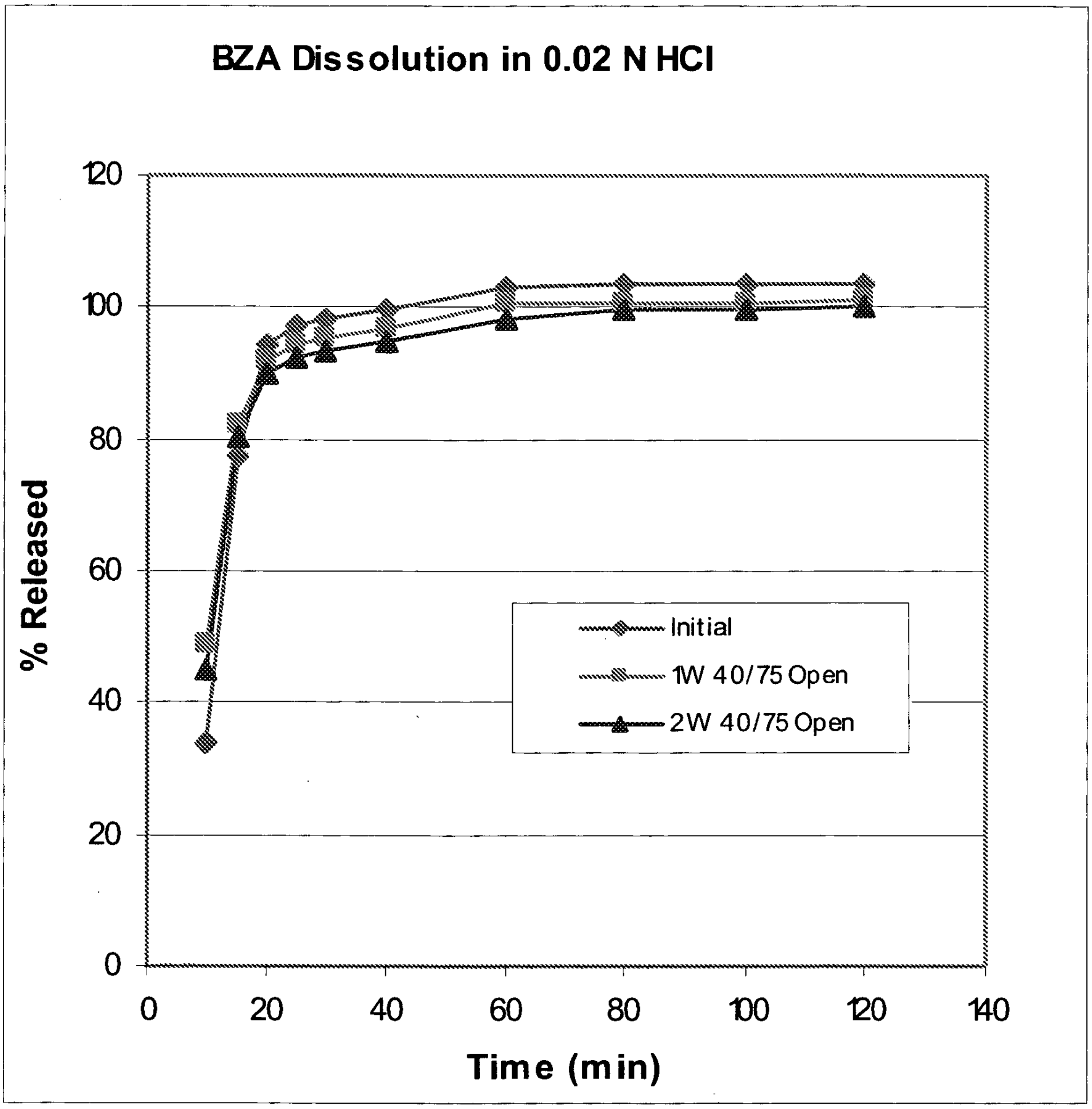
BZA Dissolution in 0.02 N HCl
% Released
Time (min)
Initial
1W 40/75 Open
2W 40/75 Open
0
20
40
60
80
100
120
140

FIGURE 9

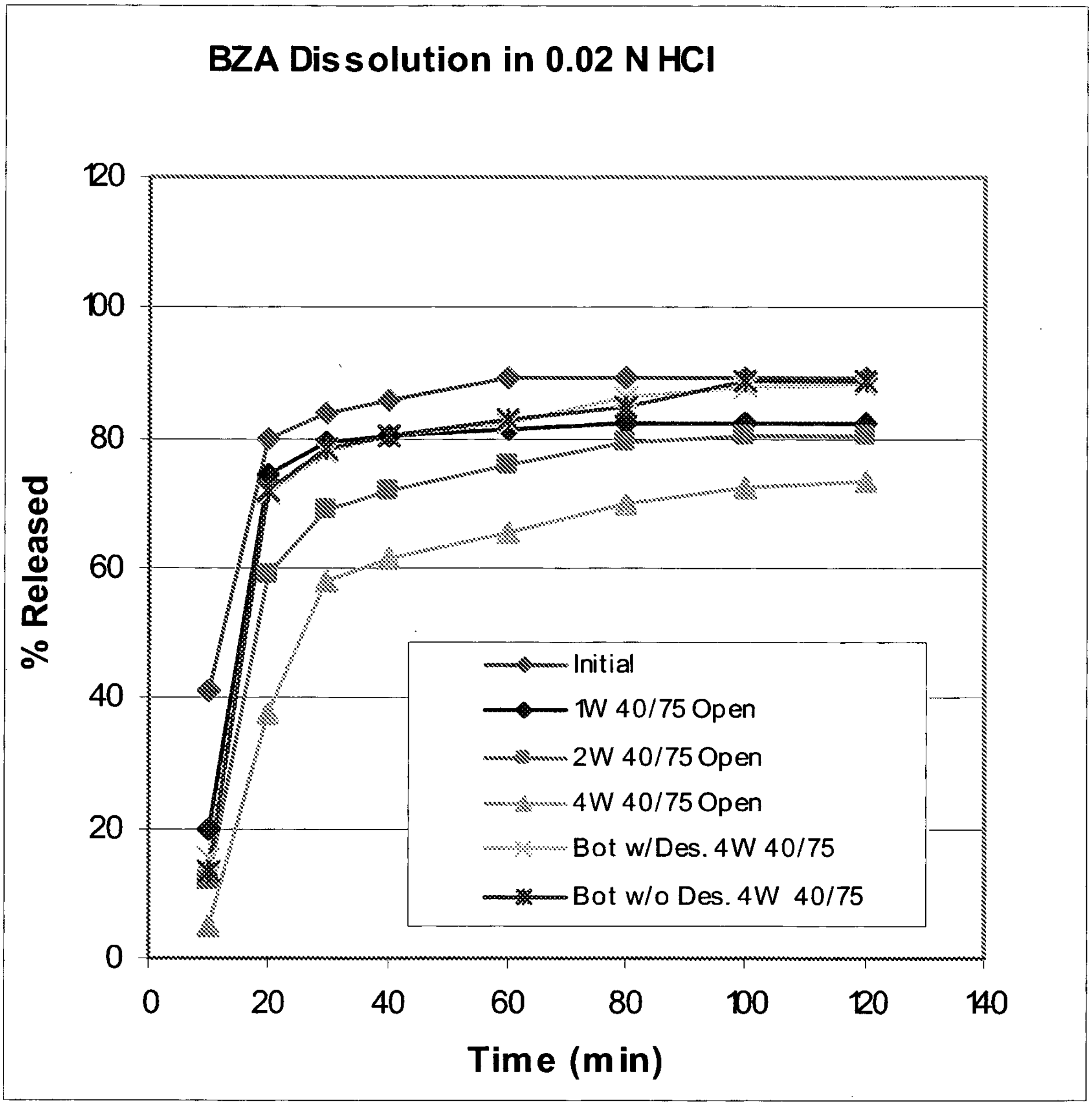
BZA Dissolution in 0.02 N HCl
% Released
Time (min)
0
20
40
60
80
100
120
140
Initial
1W 40/75 Open
2W 40/75 Open
4W 40/75 Open
Bot w/Des. 4W 40/75
Bot w/o Des. 4W 40/75

FIGURE 10

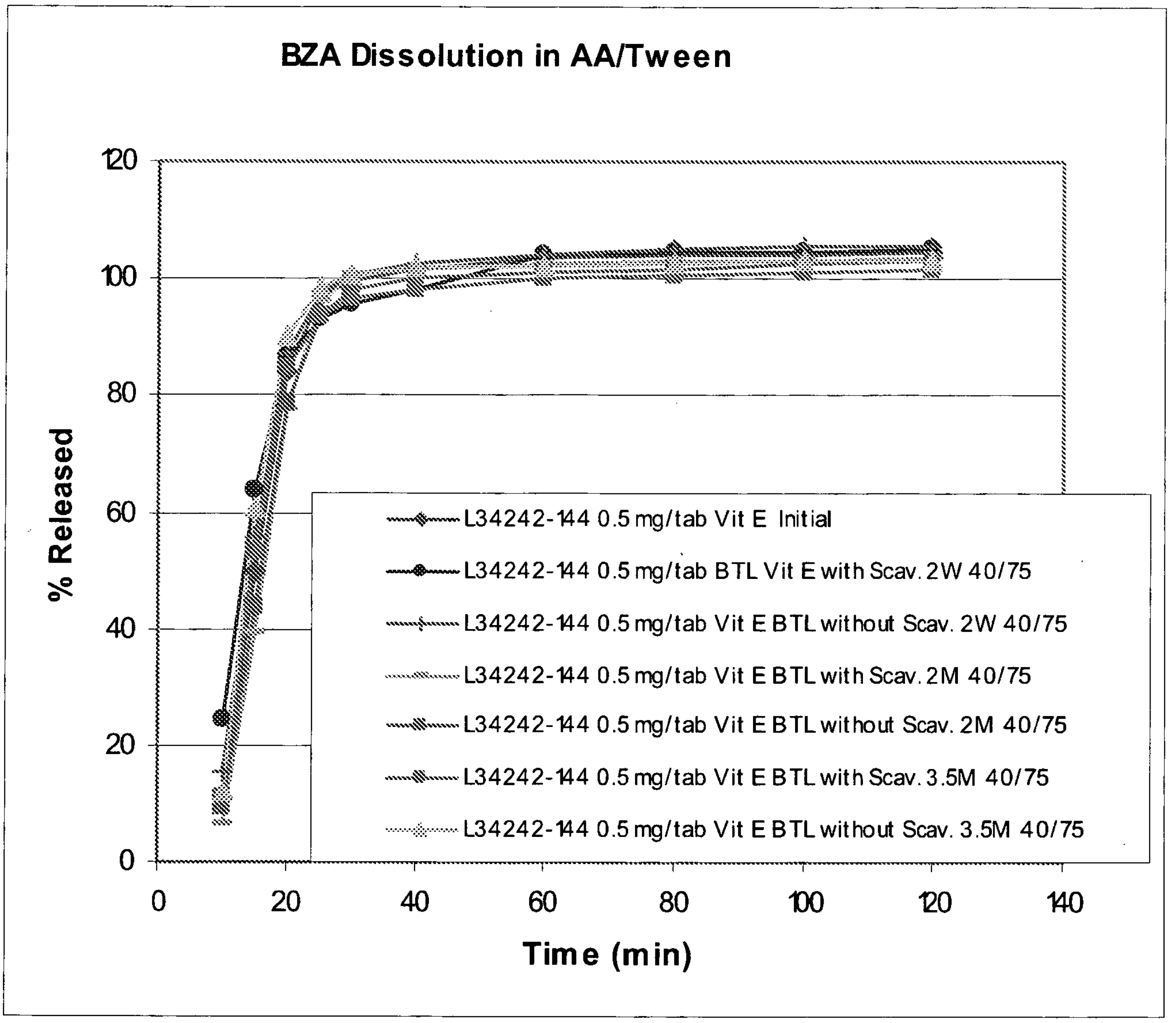
BZA Dissolution in AA/Tween
120
100
80
60
40
20
0
% Released
0
20
40
60
80
100
120
140
Time (min)
L34242-144 0.5 mg/tab Vit E Initial
L34242-144 0.5 mg/tab BTL Vit E with Scav. 2W 40/75
L34242-144 0.5 mg/tab Vit E BTL without Scav. 2W 40/75
L34242-144 0.5 mg/tab Vit E BTL with Scav. 2M 40/75
L34242-144 0.5 mg/tab Vit E BTL without Scav. 2M 40/75
L34242-144 0.5 mg/tab Vit E BTL with Scav. 3.5M 40/75
L34242-144 0.5 mg/tab Vit E BTL without Scav. 3.5M 40/75

FIGURE 11

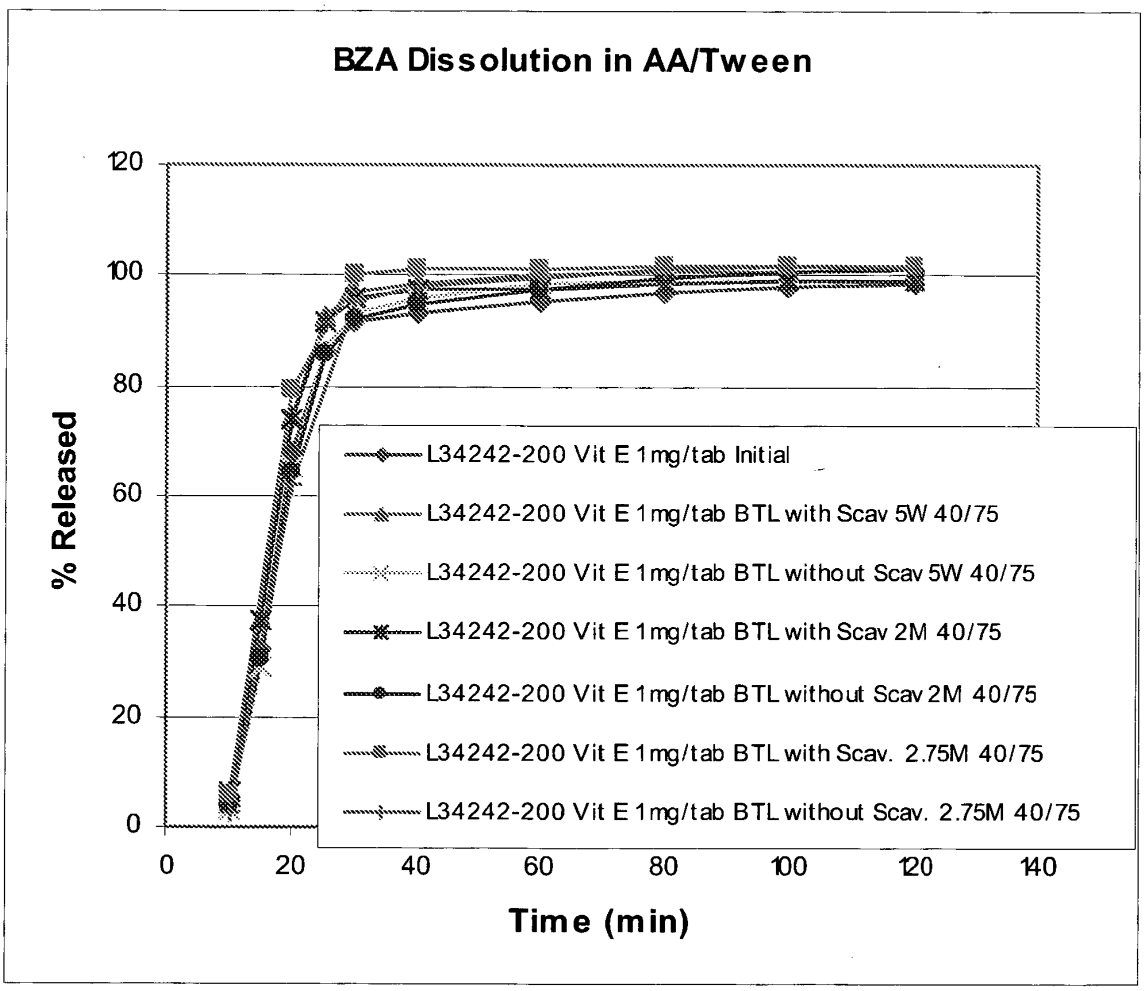
BZA Dissolution in AA/Tween
% Released
120
100
80
60
40
20
0
0
20
40
60
80
100
120
140
Time (min)
L34242-200 Vit E 1mg/tab Initial
L34242-200 Vit E 1mg/tab BTL with Scav 5W 40/75
L34242-200 Vit E 1mg/tab BTL without Scav 5W 40/75
L34242-200 Vit E 1mg/tab BTL with Scav 2M 40/75
L34242-200 Vit E 1mg/tab BTL without Scav 2M 40/75
L34242-200 Vit E 1mg/tab BTL with Scav. 2.75M 40/75
L34242-200 Vit E 1mg/tab BTL without Scav. 2.75M 40/75

FIGURE 12

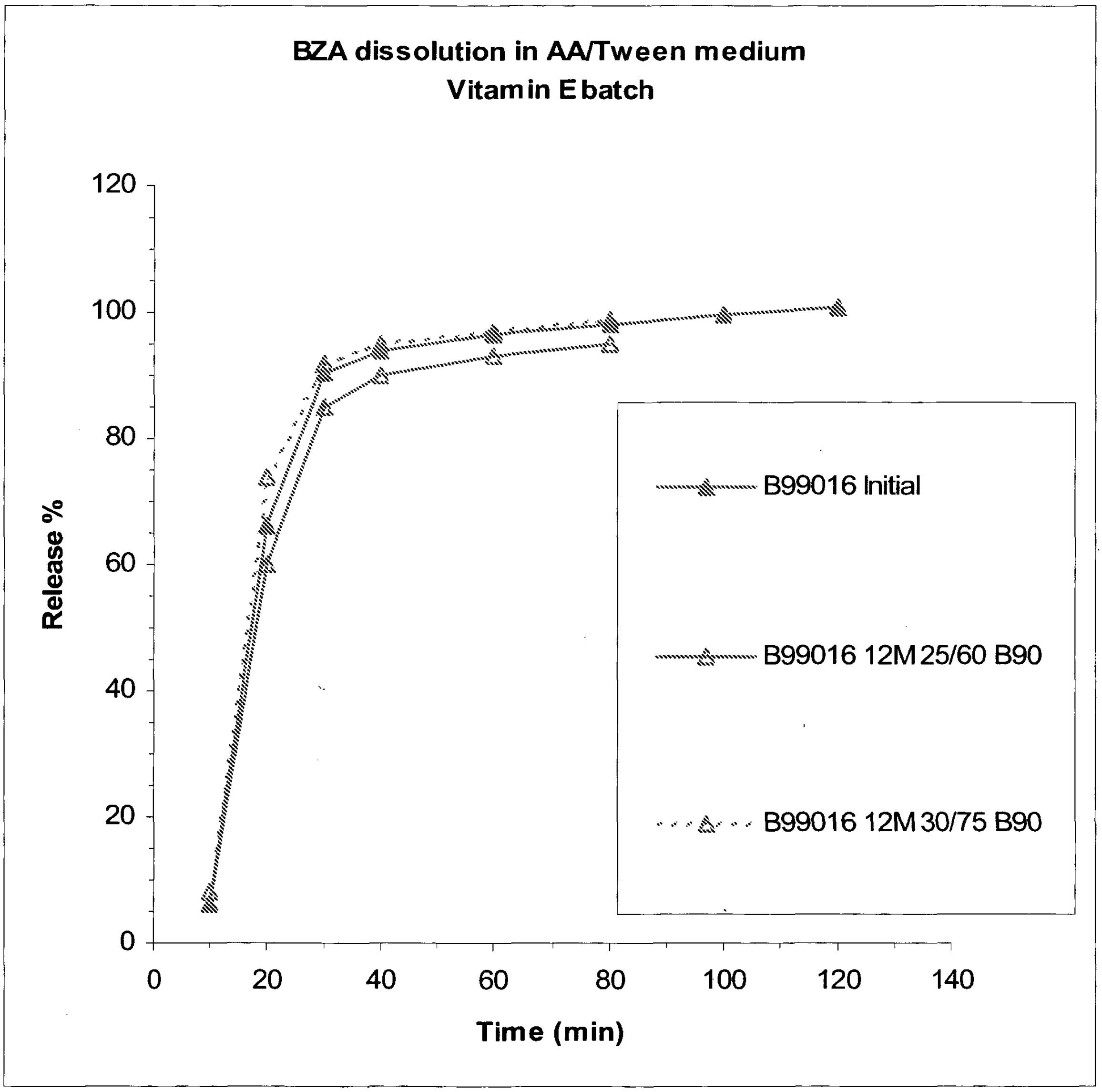
BZA dissolution in AA/Tween medium
Vitamin E batch
Release %
Time (min)
0
20
40
60
80
100
120
140
B99016 Initial
B99016 12M 25/60 B90
B99016 12M 30/75 B90

FIGURE 13

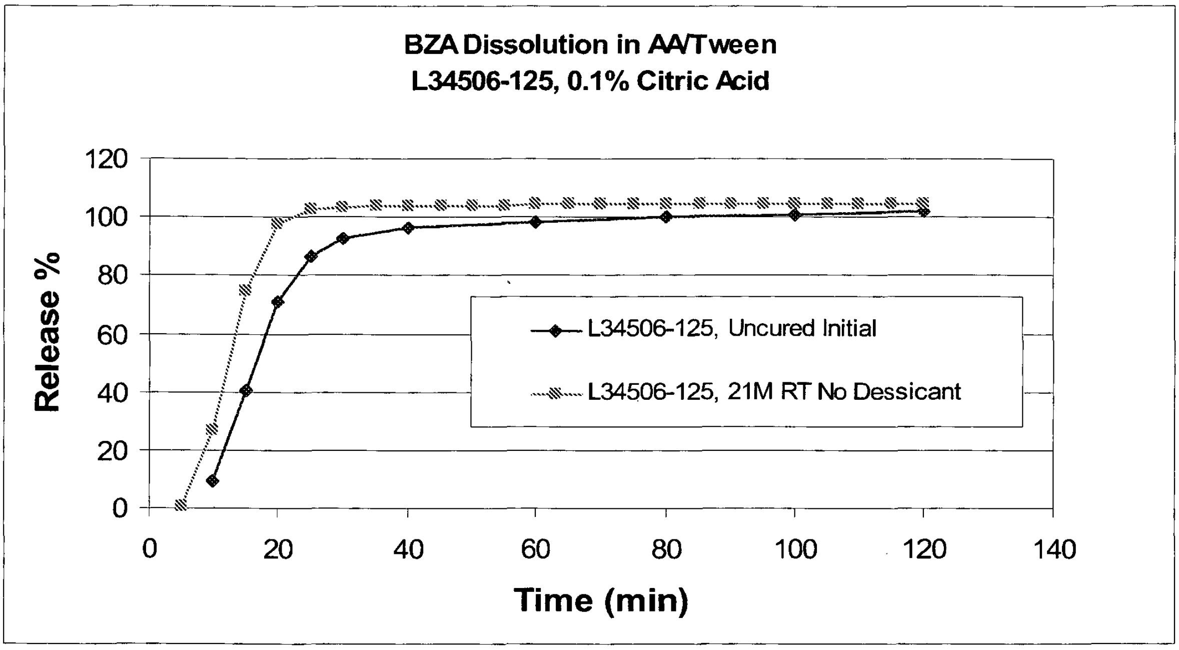
BZA Dissolution in AA/Tween
L34506-125, 0.1% Citric Acid
Release %
120
100
80
60
40
20
0
0
20
40
60
80
100
120
140
Time (min)
L34506-125, Uncured Initial
L34506-125, 21M RT No Dessicant

FIGURE 14

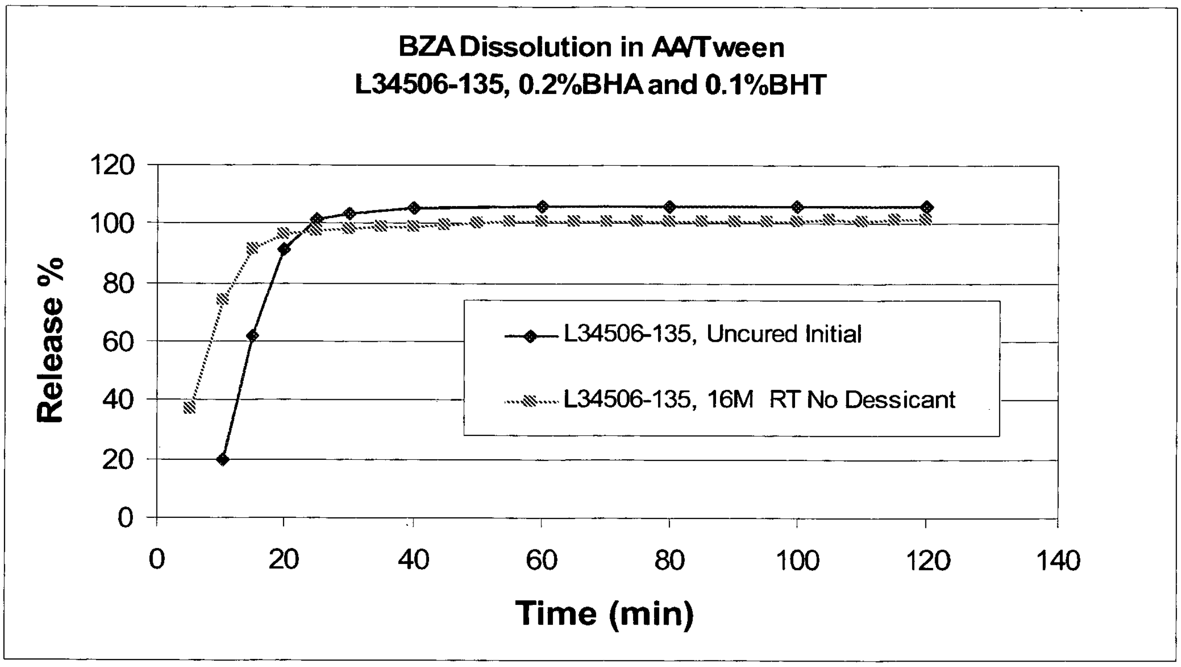
BZA Dissolution in AA/Tween
L34506-135, 0.2%BHA and 0.1%BHT
Release %
120
100
80
60
40
20
0
0
20
40
60
80
100
120
140
Time (min)
L34506-135, Uncured Initial
L34506-135, 16M RT No Dessicant

FIGURE 15

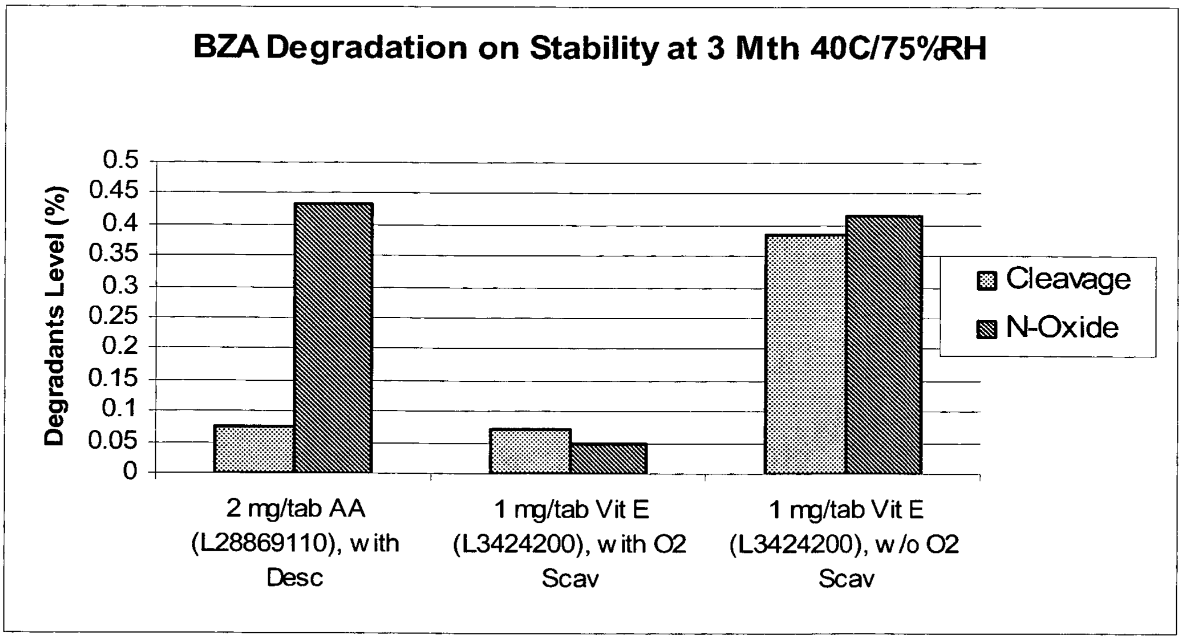
BZA Degradation on Stability at 3 Mth 40C/75%RH
Degradants Level (%)
0.5
0.45
0.4
0.35
0.3
0.25
0.2
0.15
0.1
0.05
0
Cleavage
N-Oxide
2 mg/tab AA (L28869110), with Desc
1 mg/tab Vit E (L3424200), with O2 Scav
1 mg/tab Vit E (L3424200), w/o O2 Scav

FIGURE 16

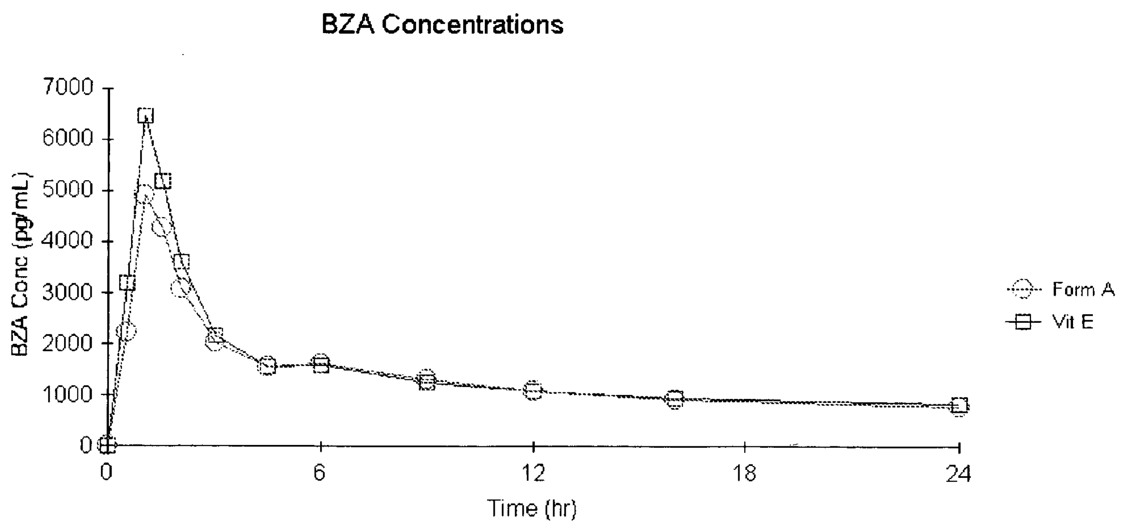
BZA Concentrations
BZA Conc (pg/mL)
7000
6000
5000
4000
3000
2000
1000
0
0
6
12
18
24
Time (hr)
Form A
Vit E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5554601 A
- US 5998402 A
- US 6479535 A
- WO 0203987 A
- US 6479535 B
- US 20070003623 A
- US 20080175905 A
- US 20080175908 A
- US 20050227965 A
- US 20050250762 A
- US 12369104 B
- US 12369315 B
- US 5210081 A
- US 2565115 A
- US 2720483 A


### Non-patent literature cited in the description


- **MILLER et al.** *J. Med. Chem.,* 2001, vol. 44, 1654-1657
- **MILLER et al.** *Drugs of the Future,* 2002, vol. 27 (2), 117-121
- **R. C. ROWE ; P. J. SHESKY.** Handbook of Pharmaceutical Excipients. Pharmaceutical Press, 2006
- **R. C. ROWE ; P. J. SHESKY.** Handbook of Pharmaceutical Excipients